# EUROPEAN PATENT SPECIFICATION

(11) **EP 2 833 880 B1**
(45) Date of publication and mention of the grant of the patent: **23.08.2017**
(21) Application number: 13773161.8
(22) Date of filing: 15.03.2013
(51) Int. Cl.: A61K 31/19, A61K 47/30, A61K 9/08, A61K 9/20, A61K 9/00

(54) **IBUPROFEN SOLID ORAL DOSAGE COMPOSITION COMPRISING A METHACRYLIC ACID COPOLYMER**
FESTE ORALE DOSIERUNGSZUSAMMENSETZUNG VON IBUPROFEN MIT EINEM METHACRYLSÄURECOPOLYMER
COMPOSITION POSOLOGIQUE SOLIDE ADMINISTRABLE PAR VOIE ORALE D'IBUPROFÈNE COMPRENANT UN COPOLYMÈRE D'ACIDE MÉTHACRYLIQUE

(30) Priority: 02.04.2012 US 201261619340 P
(43) Date of publication of application: 11.02.2015
(73) Proprietor: Pharma Seeds Create, LLC, Sanda Hyogo 669-1347 (JP)
(72) Inventor: HIBI, Toru, Sando Hyogo 669-1347 (JP); DAS, Aditya R., Foster City California 94404 (US)
(74) Representative: Wilkinson, Stephen John
(86) International application number: PCT/US2013/032199
(87) International publication number: WO 2013/151754

(56) References cited:
- EP-A1- 0 524 180
- WO-A1-01/35930
- WO-A1-2010/127346
- US-A1- 2002 076 444
- US-A1- 2002 076 444
- US-A1- 2007 196 494
- US-A1- 2008 014 268
- US-A1- 2009 011 007
- US-A1- 2009 074 867
- ANDREAS GRYCZKE ET AL: "Development and evaluation of orally disintegrating tablets (ODTs) containing Ibuprofen granules prepared by hot melt extrusion", COLLOIDS AND SURFACES. B, BIOINTERFACES, ELSEVIER, AMSTERDAM, NL, vol. 86, no. 2, 5 April 2011 (2011-04-05), pages 275-284, XP028223123, ISSN: 0927-7765, DOI: 10.1016/J.COLSURFB.2011.04.007 [retrieved on 2011-04-13]
- GRYCZKE, A. ET AL.: 'Development and evaluation of orally disintegrating tablets (ODTs) containing Ibuprofen granules prepared by hot melt extrusion' COLLOIDS AND SURFACES B: BIOINTERFACES vol. 86, 2011, pages 275 - 284, XP028223123

## Description

### INTRODUCTION

A variety of different medications, including medications available over-the-counter, have been developed to treat sore throat, laryngitis, mouth and throat ulcers, excessive mucus and other mouth and throat irritations which typically accompany common colds, influenza and other ailments.

Ibuprofen (2-(p-isobutylphenyl)propionic acid) is a non-steroidal anti-inflammatory agent (NSAID) that possesses analgesic and antipyretic activities. Ibuprofen may be used to treat pain and inflammation associated with various maladies, such as the common cold, toothache, headache, backache, menstrual cramps (Dysmennorhea), the muscular aches and pains, rheumatoid arthritis and osteoarthritis, among other types of pain, as well as in the reduction of fever.

Like other NSAIDs, ibuprofen has become widely used in prescription and over-the-counter formulations for the treatment of pain associated with both minor and chronic inflammation. Ibuprofen, however, has an unpleasant, bitter taste which limits acceptability in certain oral dosage forms. The prior art document US2007/196494 discloses a solid oral composition comprising an homogenous mixture of an AINS (meloxicam) that could also be Ibuprofen and a methacrylic acid copolymer.

### SUMMARY

Aspects of the invention include organoleptically acceptable solid oral dosage compositions of ibuprofen. Solid oral dosage compositions according to certain embodiments include ibuprofen and a methacrylic acid copolymer in an amount sufficient to make the composition organoleptically acceptable for administering in an oral cavity of a subject to deliver ibuprofen to the subject wherein the solid oral dosage composition is formulated for maintaining in the oral cavity of a subject for 15 minutes or more to deliver ibuprofen to the subject. Methods for preparing and using solid oral dosage compositions of the invention are also described.

In embodiments of the invention, organoleptically acceptable solid oral dosage compositions of ibuprofen include an amount of ibuprofen and a methacrylic acid copolymer having the formula: 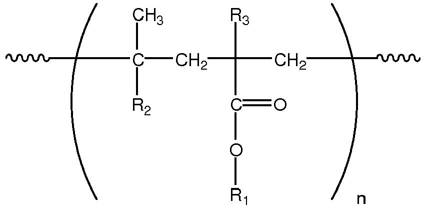 Where R₁ is ethyl, R₂ is a carboxylic acid and R₃ is -H. Solid oral dosage compositions of interest according to certain embodiments include an amount of ibuprofen which ranges from 20 mg to 200 mg and an amount of methacrylic acid copolymer which ranges from 40 mg to 400 mg. In some embodiments, solid oral dosage compositions further include one or more of a souring agent, a buffer, a diluent, a disintegrant, a lubricant and a sweetener. In certain embodiments, solid oral dosage compositions of interest consist of ibuprofen, a methacrylic acid copolymer in an amount sufficient to make the solid oral dosage composition organoleptically acceptable for dissolution in an oral cavity of a subject, a diluent, a lubricant, a souring agent, a flavoring agent, a binder, a sweetener and a buffer. In certain other embodiments, solid oral dosage compositions of interest consist of ibuprofen, a methacrylic acid copolymer in an amount sufficient to make the solid oral dosage composition organoleptically acceptable for disintegration in an oral cavity of a subject, a diluent, a disintegrant, a lubricant, a souring agent, a flavoring agent, a binder, a sweetener and a buffer.

Aspects of the invention also include methods for preparing an organoleptically acceptable solid oral dosage composition of ibuprofen. In some embodiments, methods for preparing the subject ibuprofen solid oral dosage compositions may be characterized by a first process of producing an intermediate ibuprofen-methacrylic acid copolymer granulate, which includes the active agent (i.e., ibuprofen) and a methacrylic acid copolymer, and then a second process of producing the final organoleptically acceptable solid oral dosage composition from the intermediate ibuprofen granulate. In these embodiments methods includes mixing ibuprofen with a methacrylic acid copolymer and a buffer to produce a ibuprofen-methacrylic acid copolymer composition; granulating the ibuprofen-methacrylic acid copolymer composition with water to produce a wet ibuprofen-methacrylic acid copolymer granulate; drying the wet ibuprofen-methacrylic acid copolymer granulate; milling the dried ibuprofen-methacrylic acid copolymer granulate to produce an intermediate ibuprofen-methacrylic acid copolymer granulate composition; mixing the intermediate ibuprofen-methacrylic acid copolymer granulate composition with a diluent, a binder, a lubricant, a souring agent, a sweetener and a flavoring agent; and tableting the mixture to produce an organoleptically acceptable ibuprofen-methacrylic acid copolymer solid oral dosage composition.

Aspects of the invention also include methods for using an organoleptically acceptable solid oral dosage composition of ibuprofen to treat oral-esophageal pain in a subject. In some embodiments, methods include placing the solid oral dosage composition having ibuprofen and a methacrylic acid copolymer into an oral cavity of the subject and maintaining the solid oral dosage composition in the oral cavity of the subject for an amount of time sufficient to dissolve the composition to treat the subject for oral-esophageal pain. In other embodiments, the solid oral dosage composition is disintegrated in the oral cavity by masticating the solid oral dosage composition such as chewing, biting or grinding the composition or in certain instances by sucking on the solid oral dosage composition to dissolve the composition.

### DETAILED DESCRIPTION

The claimed invention includes organoleptically acceptable solid oral dosage compositions of ibuprofen. Solid oral dosage compositions according to certain embodiments include ibuprofen and the claimed methacrylic acid copolymer in an amount sufficient to make the composition organoleptically acceptable for administering in an oral cavity of a subject to deliver ibuprofen to the subject. Methods for preparing and using solid oral dosage compositions of the invention are also described.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

As reviewed above, the present invention provides organoleptically acceptable solid oral dosage compositions of ibuprofen, as well as methods for preparing and using compositions of the invention. In further describing embodiments of the invention, organoleptically acceptable solid oral dosage compositions of ibuprofen are first reviewed in greater detail. Next, methods for preparing and using the compositions are described. Kits including one or more ibuprofen-methacrylic acid copolymer solid oral dosage compositions are also described.

### ORGANOLEPTICALLY ACCEPTABLE SOLID ORAL DOSAGE COMPOSITIONS OF IBUPROFEN

As summarized above, the claimed invention provides organoleptically acceptable solid oral dosage compositions of ibuprofen. By "organoleptically acceptable" is meant that the subject compositions are acceptable to the senses of the recipient, in particular, to the sense of taste. As described in greater detail below, organoleptically acceptable solid oral dosage compositions are those in which the unpleasant and bitter taste of ibuprofen is masked such that the ibuprofen compositions may be maintained in the oral cavity of a subject for an amount of time sufficient to dissolve or disintegrate the solid oral dosage composition. For example, the bitterness of the ibuprofen solid oral dosage composition may be reduced by 2 fold or greater, such as 3 fold or greater, such as 4 fold or greater and including 5 fold or greater, e.g., as compared to a suitable control formation that is identical to the composition but for the presence of the methacrylic acid copolymer (such as described in greater detail below). As such, the bitterness of ibuprofen solid oral dosage compositions provided by the invention may be 50% or less as compared to unmasked ibuprofen, such as 10% or less as compared to unmasked ibuprofen.

In certain embodiments, the bitterness of compositions is measured on a bitterness rating scale of 1-5, where compositions having a bitterness rating of 1 are not bitter and compositions having a bitterness rating of 5 are extremely bitter. In these embodiments, a bitterness rating of 1 has a slightly metallic flavor, a bitterness rating of 2 has a moderate metallic flavor, a bitterness rating of 3 has a sour to metallic flavor, a bitterness rating of 4 has a bitter flavor and a bitterness rating of 5 has an extremely bitter flavor. Where unmasked ibuprofen possesses a bitterness rating of 5, solid oral dosage compositions of the present invention may have a bitterness rating of 3 or less, such as 2 or less, and including a bitterness rating of 1. Bitterness may be determined using any convenient protocol. For example, bitterness may be determined using a blind taste test with healthy volunteers using appropriate controls (e.g., pure sugar).

In embodiments of the invention, ibuprofen solid oral dosage compositions having one or more methacrylic acid copolymers are provided. By "solid oral dosage composition" is meant a solid medicated composition which is configured to be administered in an oral cavity of a subject and is maintained (e.g., dissolved, chewed, grinded, etc.) over 15 minutes or more to release ibuprofen into the oral cavity. As such, solid oral dosage compositions of the invention may be tablets, chewable tablets, orally disintegrating tablets, troches or lozenges, including solid compositions which are produced by tableting or pelleting compressed powders, granules or pastes.

In certain embodiments, solid oral dosage compositions are troches. The term "troche" is used in its conventional sense to refer to a solid which is configured to dissolve in the oral cavity over a predetermined amount of time while retaining its shape during dissolution. As such, where solid oral dosage compositions of the invention are troches, the solid oral dosage composition is configured to be entirely dissolved in the oral cavity. Depending on the type of ailment, size of the troche, physiology of the subject and desired treatment times, troches of the invention may be slow dissolution troches or fast dissolution troches. In some instances, solid oral dosage compositions are slow dissolution troches, such as where the amount of time required for the troche to dissolve in the oral cavity is 15 minutes or more. Since troches are formulated to be dissolved in the oral cavity while retaining their shape, the release of ibuprofen during dissolution may vary. For example, ibuprofen-methacrylic acid copolymer troches of the present claimed invention provide a sustained release of ibuprofen. By "sustained release" is meant that the troche is formulated to provide for constant and continuous delivery of ibuprofen over the entire time troche is maintained in the oral cavity, such as 15 minutes or longer, such as 30 minutes or longer, and including 60 minutes or longer. In other instances, ibuprofen-methacrylic acid copolymer troches of the invention may provide a pulsatile release of ibuprofen. By "pulsatile release" is meant that the troche is formulated to release ibuprofen into the oral cavity incrementally (e.g., at discrete times) during dissolution, such as every 1 minute of dissolution, such as every 2 minutes of dissolution, such as every 5 minutes of dissolution and including every 10 minutes of dissolution. In other instances, ibuprofen may be delivered to the oral cavity after certain percentages of the solid oral dosage composition is dissolved. For example, ibuprofen may be delivered after every 10% of the solid oral dosage composition is dissolved in the oral cavity, such as every 15% of the solid oral dosage composition is dissolved, such as every 20% of the solid oral dosage composition is dissolved, such as every 25% of the solid oral dosage composition is dissolved, such as every 30% of the solid oral dosage composition is dissolved and including after every 33% or of the solid oral dosage composition is dissolved in the oral cavity. In yet other instances, ibuprofen-methacrylic acid copolymer troches of the invention may be formulated to release a large amount of ibuprofen immediately upon contact with the oral cavity (such as to provide an acute reduction in pain), such as 50% or more, such as 60% or more, such as 70% or more and including 90% or more of the ibuprofen is released immediately upon contact with the oral cavity.

In other embodiments, solid oral dosage compositions are chewable tablets. The term "chewable tablet" is used in its conventional sense to refer to a tablet which is formulated to be disintegrated in an oral cavity by mastication, such as by chewing or grinding with the teeth or gums. Since chewable tablets are formulated to be disintegrated by mastication, chewable tablets of the invention have a hardness which is safe for chewing without any adverse effects on the teeth or gums during mastication. In certain instances, solid oral dosage compositions are formulated as chewable tablets by further including one or more disintegrants in the ibuprofen-methacrylic acid copolymer solid oral dosage composition, as described in greater detail below.

In yet other embodiments, solid oral dosage compositions are orally-disintegrating tablets. The term "orally disintegrating tablet" is used in its conventional sense to refer to a tablet which is formulated to be break apart into small fragments when placed in the oral cavity. Fragmentation or break down of orally disintegrating tablets may be aided by mastication, but the orally disintegrating tablets may be formulated to break down into fragments with little or no chewing or grinding by teeth or gums. As discussed in greater detail below, solid oral dosage compositions may be formulated as orally disintegrating tablets by including one or more disintegrants in the ibuprofen-methacrylic acid copolymer solid oral dosage composition.

Depending on the location in the oral cavity where the solid oral dosage composition is administered, compositions of the invention may be any convenient shape and size. For example, solid oral dosage compositions may be cubes, capsules, disks, spheres, thin strips, rectangular prisms, triangular prisms, hexagonal prisms, cylinders, among other shapes. The size of the solid oral dosage composition may vary depending on the physiology of the subject (e.g., the size of the oral cavity), the dosage of ibuprofen and the duration of treatment, as desired. As such, the size of the composition may range from 0.5 to 5 cm³, such as 1.0 to 5 cm³, such as 1.5 to 4.5 cm³, such as 2.0 to 4 cm³, such as 2.5 to 3.5 cm³, and including 2 to 3 cm³. As described in greater detail below, in some embodiments, solid oral dosage compositions of interest may be in the form of tablets produced by dry compression of a granulated composition. Depending on the method of administration (e.g., chewing, dissolution), as described in greater detail below, the hardness of the solid oral dosage composition may vary, ranging from 2 to 25 kP (kilopond) per cm², such as 3 to 22 kP per cm², such as 5 to 20 kP per cm², such as 5 to 15 kP per cm², such as 5 to 12 kP per cm² and including such as 5 to 10 kP per cm².

Aspects of the invention include a solid oral dosage composition having ibuprofen and a methacrylic acid copolymer. The term "ibuprofen" is used in its conventional sense to refer to 2-(4-isobutylphenyl)-propionic acid and pharmaceutically acceptable salts thereof, including but not limited to arginine, lysine, histidine, sodium (Na⁺), potassium (K⁺), lithium (Li⁺), magnesium (Mg²⁺), calcium (Ca²⁺), zinc (Zn²⁺) and aluminum (Al³⁺) salts, as well as other salts. Furthermore, solid oral dosage compositions according to embodiments of the invention may include ibuprofen as a racemic mixture or as a pure enantiomer, such as the R or L enantiomers. However, the subject solid oral dosage compositions are not merely limited to ibuprofen and in certain embodiments, may include one or more different propionic acid derivatives in place of or in addition to ibuprofen. For example, solid oral dosage compositions of the present invention may include, but is not limited to, ibuprofen, naproxen, benoxaprofen, naproxen sodium, flurbiprofen, fenoprofen, fenbuprofen, ketoprofen, indoprofen, pirprofen, carpofen, oxaprofen, pranoprofen, microprofen, tioxaprofen, suproprofen, alminoprofen, tiaprofenic acid, fluprofen, bucloxic acid, or any other anti-inflammatory analgesic propionic acid derivative having a free -CH(CH₃)COOH or -CH₂ CH₂ COOH group or a pharmaceutically acceptable salt group, such as -CH(CH₃)COO-Na⁺ or CH₂ CH₂ COO-Na⁺, attached directly or via a carbonyl functionality to an aromatic ring system.

The amount of ibuprofren in solid oral dosage compositions of the invention may vary, as long as the amount of ibuprofen is sufficient to treat the ailment of the subject in need thereof, as described in greater detail below. For example, the amount of ibuprofen in subject compositions may range from 5 to 800 mg, such as 10 to about 500 mg, such as 20 to 400 mg, such as 25 to 350 mg, such as 30 to 300 mg, such as 40 to 250 mg and including 40 to 200 mg. As such, the percent by weight of ibuprofen in solid oral dosage compositions of interest may vary, ranging from 0.3% to 75% by weight ibuprofen, such as 1% to 70% by weight ibuprofen, such as 5% to 50% by weight ibuprofen, such as 10% to 40% by weight ibuprofen, such as 15% to 35% by weight ibuprofen and including 15% to 25% by weight ibuprofen.

As reviewed above, solid oral dosage compositions of the present invention also include a methacrylic acid copolymer. By "methacrylic acid copolymer" is meant the class of polymeric compounds described by the formula: 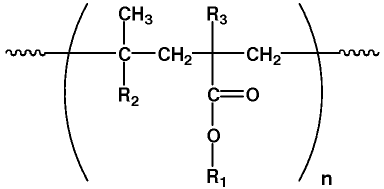 wherein R₁ is ethyl, R₂ is carboxylic acid and R₃ is H. In some embodiments, the methacrylic acid copolymer is a Eudragit^{®} methacrylic acid copolymer. The term "Eudragit^{®} methacrylic acid copolymer" is used in its conventional sense to refer to copolymers derived from esters of acrylic and methacrylic acid. In embodiments of the invention, Eudragit^{®} polymers may be methacrylic acid copolymers (e.g., functional group being carboxylic acid). In certain embodiments, the Eudragit^{®} polymer is a methacrylic acid Eudragit^{®} polymer, such as Eudragit^{®} L100 or Eudragit^{®} L100-55. In the claimed invention, the methacrylic acid copolymer is present in solid oral dosage compositions in an amount sufficient to make the composition organoleptically acceptable for administering in an oral cavity of a subject. In some embodiments, solid oral dosage compositions include a matrix having methacrylic acid copolymer-ibuprofen polyelectrolyte complexes where ibuprofen is complexed with methacrylic acid copolymer by ionic interactions. In these embodiments, the bitter taste of ibuprofen is sufficiently masked by the complexation of ibuprofen with methacrylic acid copolymer. As such, the subject compositions will remain organoleptically acceptable substantially throughout the entire time the composition is maintained in the oral cavity. In other words, for as long as any amount of the subject compositions is present in the oral cavity, the bitterness of ibuprofen is masked (e.g., as described below). Likewise, compositions according to these embodiments are organoleptically acceptable irrespective of the route of administration (e.g., dissolved, masticated, disintegrated without mastication, etc. as described below). For example, since the methacrylic acid copolymer-ibuprofen polyelectrolyte complexes are resistant to shearing or disintegrating forces (e.g., during mastication or dissolution), the bitterness taste masking by the methacrylic acid copolymer-ibuprofen polyelectrolyte complexes remains the same whether the solid oral dosage composition is chewed, dissolved in the oral cavity or orally disintegrated. In certain embodiments, the matrix of the subject solid oral dosage compositions includes methacrylic acid copolymer-ibuprofen nanoparticles.

Accordingly, the amount of methacrylic acid copolymer in compositions of interest may vary, depending on the dosage of ibuprofen, as described above. For example, the amount of methacrylic acid copolymer present may range from 5 to 1200 mg, such as 10 to about 1000mg, such as 20 to 900 mg, such as 25 to 800 mg, such as 25 to 700 mg, such as 30 to 500 mg and including 40 to 400 mg. As such, the percent by weight of methacrylic acid copolymer in solid oral dosage compositions of interest may vary, ranging from 0.3% to 75% by weight methacrylic acid copolymer, such as 1% to 70% by weight methacrylic acid copolymer, such as 5% to 50% by weight methacrylic acid copolymer, such as 10% to 40% by weight methacrylic acid copolymer, such as 15% to 35% by weight methacrylic acid copolymer and including 15% to 25% by weight methacrylic acid copolymer.

The mass ratio of ibuprofen to methacrylic acid copolymer in solid oral dosage compositions as claimed ranges between 1:1 and 1:3

In some embodiments, the solid oral dosage composition may further include a souring agent. By souring agent is meant a component that includes one or more compositions sufficient for providing a sour taste when the composition is placed in an oral cavity of a subject. In certain embodiments where it is desired to dissolve the subject solid oral dosage composition (e.g., troches, lozenges) without mastication (i.e, chewing, grinding or biting), the souring agent may be one or more compositions which help to prevent biting or chewing before dissolution of the solid oral dosage composition in the oral cavity. In yet other embodiments, the souring agent may be one or more compositions which help to prevent swallowing of the solid oral dosage composition.

In some embodiments, the souring agent is an organic acid. The term "organic acid" is used in its conventional sense to refer to organic compounds having at least weak acidity (i.e., dissociation in water) and may include, but are not limited to: glycolic acid, lactic acid, methyl lactic acid, polycarboxylic acids, malic acid, citric acid, tartronic acid, tartaric acid, asparaginic acid, succinic acid as well as amino acids. Amino acids of interest include, but are not limited to: glycine, alanine, valine, leucine, isoleucine, serine, threonine, cysteine, cystine, methionine, aspartic acid, asparagine, glutamic acid, glutamine, arginine, lysine, 5-hydroxylysine, histidine, phenylalanine, tyrosine, tryptophan, 3-hydroxyproline, 4-hydroxyproline, proline, homocysteine, homocystine, homoserine, ornithine, citrulline, creatine, asparaginic acid, 3-aminopropanoic acid, theanine, 2-aminobutanoic acid, 4-aminobutanoic acid, 2-amino-2-methylpropanoic acid, 2-methyl-3-aminopropanoic acid, 2,6-diaminopimelic acid, 2-amino-3-phenylbutanoic acid, phenylglycine, canavanine, canaline, 4-hydroxyarginine, 4-hydroxyornithine, homoarginine, 4-hydroxyhomoarginine, β-lysine, 2,4-diaminobutanoic acid, 2,3-diaminopropanoic acid, 2-methylserine, 3-phenylserine betaine, sulfur-containing amino acids, such as taurine, cysteinesulfinic acid, methionine sulfoxide and methionine sulfone.

The amount of souring agent present in solid oral dosage claimed compositions of the invention may vary, depending on the amount of ibuprofen, as described above as well as the size of the composition and subject to which the composition is applied. In some embodiments, the amount of souring agent in solid oral dosage compositions of interest may range from 2 to 500 mg, such as 5 to about 250 mg, such as 10 to 200 mg, such as 12 to 150 mg, such as 15 to 100 mg, such as 20 to 75 mg and including 5 to 50 mg. Depending on the amount of ibuprofen present in the composition, the mass ratio of ibuprofen to souring agent may vary, ranging from 0.2 to 5, such as from 0.5 to 4, such as from 1 to 3, and including 1 to 2. In other embodiments, the mass ratio of souring agent to ibuprofen may vary, ranging from 0.2 to 5, such as from 0.5 to 4, such as from 1 to 3, and including 1 to 2. For example, the mass ratio of ibuprofen to souring agent may be 0.8. As such, the percent by weight of souring agent in solid oral dosage compositions of interest may vary, ranging from 0.1 % to 75% by weight souring agent, such as 1% to 70% by weight souring agent, such as 5% to 50% by weight souring agent, such as 10% to 40% by weight souring agent, such as 15% to 35% by weight souring agent and including 15% to 25% by weight souring agent.

In some embodiments, solid oral dosage compositions of the claimed invention include two or more souring agents. For example, solid oral dosage compositions of the invention may include two or more organic acids. For example, compositions may include tartaric acid and citric acid. Where compositions include more than one souring agents, the mass percentage of each souring agent may vary, ranging from 1% or more of the total mass of the solid oral dosage composition, such as 2% or more, such as 5% or more, such as 10% or more, including 25% or more of the total mass of the solid oral dosage composition.

In certain instances, solid oral dosage claimed compositions of the invention include tartaric acid and citric acid. Where the composition includes a combination of tartaric acid and citric acid, the mass ratio of tartaric acid and citric acid may vary, ranging between 1:1 and 1:10, such as 1:2, such as 1:3, such as 1:4, such as 1:5 and including 1:10. In other instances, the mass ratio of citric acid and tartaric acid may vary, ranging between 1:1 and 1:10, such as 1:2, such as 1:3, such as 1:4, such as 1:5 and including 1:10.

In some embodiments, solid oral dosage compositions may further include one or more sweeteners. By sweetener is meant one or more compositions that provides a sweet taste when the solid oral dosage composition is placed in an oral cavity of a subject. In certain instances, the sweetener may be one or more compositions which help to sweeten the bitter taste of ibuprofen or sweeten the sour taste of a souring agent in the solid oral dosage composition. In certain embodiments where it is desired to disintegrate the subject solid oral dosage composition by mastication (i.e, chewing, grinding or biting), the sweetener may be one or more compositions which help to encourage biting or chewing of the solid oral dosage composition. For example, one or more sweeteners which encourage biting or chewing may be included in ibuprofen-methacrylic acid copolymer solid oral dosage compositions to formulate the composition as a chewable tablet.

Where solid oral dosage compositions include a sweetener, the sweetener may be a natural sugar or sugar alcohol, such as for example glucose, fructose, glycerol, sorbitol, or sucrose. In some instances, the sweetener is an artificial sweetener, such as for example a sugar substitute, including but not limited to stevia, aspartame, Magnasweet, sucralose, neotame, acesulfame potassium, and saccharin.

The amount of sweetener present in solid oral dosage compositions of the invention may vary, depending on the amount of ibuprofen, as described above as well as the size of the solid oral dosage composition, any souring agent added and the subject to which the composition is administered. In some embodiments, the amount of sweetener in solid oral dosage compositions of interest may range from 2 to 500 mg, such as 5 to about 250 mg, such as 10 to 200 mg, such as 12 to 150 mg, such as 15 to 100 mg, such as 20 to 75 mg and including 5 to 50 mg. Depending on the amount of ibuprofen present in the composition, the mass ratio of ibuprofen to sweetener may vary, ranging from 0.2 to 5, such as from 0.5 to 4, such as from 1 to 3, and including 1 to 2. In other embodiments, the mass ratio of sweetener to ibuprofen may vary, ranging from 0.2 to 5, such as from 0.5 to 4, such as from 1 to 3, and including 1 to 2. For example, the mass ratio of ibuprofen to sweetener may be 0.3. As such, the percent by weight of sweetner in solid oral dosage compositions of interest may vary, ranging from 0.1% to 75% by weight sweetner, such as 1% to 70% by weight sweetner, such as 5% to 50% by weight sweetner, such as 10% to 40% by weight sweetner, such as 15% to 35% by weight sweetner and including 15% to 25% by weight sweetner.

In some embodiments, solid oral dosage compositions may have a sweet flavor, a sour flavor or a blend of sweet and sour flavor. The amount of sweetness or sourness of subject solid oral dosage compositions may vary. For example, where it is desired to have a sweet tasting solid oral dosage composition (e.g., to encourage disintegration by mastication), the composition may be formulated to have an amount of sweetener which exceeds the amount of souring agent. In these embodiments, the mass ratio of sweetener to souring agent ranges, depending on the type of sweetener and souring agent as described above and may be from 10 to 0.1, such as from 10 to 0.5, such as from 10 to 1, such as 5 to 1, such as 4 to 1, such as 3 to 1, and including 2 to 1. In other embodiments, where it is desired to have a sour tasting solid oral dosage composition (e.g., to discourage mastication and encourage dissolution), the composition may be formulated to have an amount of souring agent which exceeds the amount of sweetener. In these embodiments, the mass ratio of souring agent to sweetener ranges, depending on the type of souring agent and sweetener as described above, from 10 to 0.1, such as from 10 to 0.5, such as from 10 to 1, such as 5 to 1, such as 4 to 1, such as 3 to 1, and including 2 to 1. In yet other instances, the solid oral dosage composition may be a blend of sweet tasting and sour tasting.

In certain embodiments, solid oral dosage claimed compositions of the invention include two or more sweeteners. For instance, solid oral dosage compositions of the invention may include two or more natural sugars or sugar alcohols. In other instances, compositions may include a natural sugar or sugar alcohol and an artificial sugar. In yet other instances, solid oral dosage compositions may include two or more artificial sugars. Where solid oral dosage compositions include more than one sweetener, the mass percentage of each sweetener may vary, ranging from 1 % or more of the total mass of the solid oral dosage composition, such as 2% or more, such as 5% or more, such as 10% or more, including 25% or more of the total mass of the solid oral dosage composition.

In certain instances, solid oral dosage compositions of the invention include a glycyrrhizin based sweetener, e.g., a monoammonium glycyrrhizinate (MAG) commercialized under the trademark Magnasweet^{™}, and sucralose. The term glycyrrhizin is used in its conventional sense to refer to the triterpenoid saponin glycoside of glycyrrhizic acid which is odorless and has a sweet flavor. Where the composition includes a combination of a glycyrrhizin based sweetener and sucralose, the mass ratio of the glycyrrhizin based sweetener and sucralose may vary, ranging between 1:1 and 1:10, such as 1:2, such as 1:3, such as 1:4, such as 1:5 and including 1:10. In other instances, the mass ratio of sucralose and glycyrrhizin based sweetener may vary, ranging between 1:1 and 1:10, such as 1:2, such as 1:3, such as 1:4, such as 1:5 and including 1:10.

As described above, in certain embodiments, solid oral dosage claimed compositions are formulated for disintegration in an oral cavity (e.g., by mastication). Depending on the desired method of disintegration (i.e., mastication, oral disintegration without mastication), solid oral dosage compositions may include one or more disintegrants. The term "disintegrants" is used in its conventional sense to refer to agents which promote the breakup of the solid oral dosage composition into smaller fragments in the oral cavity to thereby increase the available surface area of solid oral dosage composition particles and to promote release of ibuprofen into the oral cavity. Disintegrants may include, but are not limited to pre-gelatinized starch, camellose calcium, hydrated silicon dioxide, croscarmellose sodium, microcrystalline cellulose, low substituted hydroxypropylcellulose (L-HPC), corn starch, potato starch, partly pre-gelatinized starch and crospovidone, among other disintegrants. The amount of disintegrant present in solid oral dosage compositions of the invention may vary, depending on the desired method of oral disintegration, as well as the size of the solid oral dosage composition. In some embodiments, the amount of disintegrant in solid oral dosage compositions of interest may range from 0 to 500 mg, such as 5 to about 450 mg, such as 10 to 400 mg, such as 25 to 300 mg and including 50 to 250 mg.

In some embodiments, solid oral dosage compositions may further include one or more buffers. The term buffer is used in its conventional sense to refer to a compound which helps to stablize (i.e., maintain) the pH of the composition, such as for example during dissolution of the solid oral dosage composition in an oral cavity of a subject. In some embodiments, the buffer in solid oral dosage compositions of the invention is a salt. Buffer salts may include, but are not limited to sodium citrate, sodium acetate, sodium phosphate, sodium tartrate, sodium succinate, sodium maleate, magnesium acetate, magnesium citrate, magnesium phosphate, ammonium acetate, ammonium citrate, ammonium phosphate, among other salts. In certain embodiments, solid oral dosage compositions of interest include sodium citrate.

The amount of buffer present in solid oral dosage compositions of the invention may vary, depending on the amount of ibuprofen, as described above as well as the size of the solid oral dosage composition and subject to which the solid oral dosage composition is administered. In some embodiments, the amount of buffer in solid oral dosage compositions of interest may range from 2 to 500 mg, such as 5 to about 250 mg, such as 10 to 200 mg, such as 12 to 150 mg, such as 15 to 100 mg, such as 20 to 75 mg and including 5 to 50 mg. Depending on the amount of ibuprofen present in the composition, the mass ratio of ibuprofen to buffer may vary, ranging from 0.1 to 5, such as 0.2 to 5, such as from 0.5 to 4, such as from 1 to 3, and including 1 to 2. In other embodiments, the mass ratio of buffer to ibuprofen may vary, ranging from 0.2 to 5, such as from 0.5 to 4, such as from 1 to 3, and including 1 to 2. For example, the mass ratio of ibuprofen to buffer may be 0.2. As such, the percent by weight of buffer in solid oral dosage compositions of interest may vary, ranging from 0.1% to 75% by weight buffer, such as 1% to 70% by weight buffer, such as 5% to 50% by weight buffer, such as 10% to 40% by weight buffer, such as 15% to 35% by weight buffer and including 15% to 25% by weight buffer.

In some embodiments, the solid oral dosage composition may further include one or more diluents. Diluents in solid oral dosage compositions of the invention may include one or more sugars, such as for example, white sugar, powder sugar, lactose, fructose, starch syrup, reduced malt sugar, D- mannitol, D-sorbitol, and sucrose. For example, in certain embodiments, the diluent is xylitol.

The amount of diluent present in solid oral dosage compositions of the invention may vary, depending on the amount of ibuprofen, as described above as well as the size of the composition and final concentration of ibuprofen desired. In some embodiments, the amount of diluent in solid oral dosage compositions of interest may be 20 mg or greater, such as 50 mg or greater, such as 100 mg or greater, such as 150 mg or greater, such as 250 mg or greater, and including 500 mg or greater. For example, the amount of diluent present in solid oral dosage compositions of interest may be 250 mg or greater. Depending on the amount of ibuprofen present in the composition, the ratio by mass of diluent to ibuprofen may vary, ranging from 0.5 to 25, such as 1to 25, such as from 1.5 to 20, and including 2.5 to 10. As such, the percent by weight of diluent in solid oral dosage compositions of interest may vary, ranging from 1% to 75% by weight diluent, such as 2% to 70% by weight diluent, such as 5% to 50% by weight diluent, such as 10% to 40% by weight diluent, such as 15% to 35% by weight diluent and including 15% to 25% by weight diluent.

In some embodiments, the solid oral dosage composition may further include one or more lubricants. Lubricants in solid oral dosage compositions of the invention may include, but is not limited to magnesium stearate, talc, stearic acid, sucrose fatty acid ester. For example, in certain embodiments, the lubricant is magnesium stearate.

The amount of lubricant present in solid oral dosage compositions of the invention may vary, depending on the amount of ibuprofen, as described above as well as the size of the solid oral dosage composition desired. In some embodiments, the amount of lubricant in solid oral dosage compositions of interest may be 1 mg or greater, such as 2 mg or greater, such as 5 mg or greater, such as 10 mg or greater, such as 25 mg or greater, such as 50 mg or greater, and including 100 mg or greater. For example, the amount of lubricant present in solid oral dosage compositions of interest may be 4 mg or greater. Depending on the amount of ibuprofen present in the solid oral dosage composition, the ratio by mass of ibuprofen to lubricant may vary, ranging from 0.1 to 100, such as 0.2 to 100, such as from 0.5 to 80, such as from 1 to 60, and including 1 to 40. As such, the percent by weight of lubricant in solid oral dosage compositions of interest may vary, ranging from 0.1 % to 75% by weight lubricant, such as 1% to 70% by weight lubricant, such as 2% to 50% by weight lubricant, such as 5% to 40% by weight lubricant, such as 10% to 35% by weight lubricant and including 15% to 25% by weight lubricant.

Solid oral dosage compositions of the invention may also further include one or more flavoring agents. In certain embodiments, compositions may have a citrus flavor, such as for example to correspond to the souring agent described above. For example, flavoring agents may include lemon, lime, lemon-lime, orange, grapefruit and tangerine. However, solid oral dosage compositions of the present invention are not limited to citrus flavors and may have any desired flavor, such as for example menthol, WS-23, apple, grape, cherry and chocolate, among other flavors.

Depending on the amount of ibuprofen present, the amount of flavoring agent present in solid oral dosage compositions of interest may vary. In certain embodiments, the amount of flavoring agent present is an amount that is sufficient to produce the desired flavor in the composition when placed in an oral cavity of a subject. As such, the amount of flavoring agent may 2 mg or greater, such as 5 mg or greater, such as 10 mg or greater, such as 25 mg or greater, such as 50 mg or greater, such as 100 mg or greater and including 250 mg or greater. As such, the percent by weight of flavoring agent in solid oral dosage compositions of interest may vary, ranging from 0.1% to 75% by weight flavoring agent, such as 1% to 70% by weight flavoring agent, such as 5% to 50% by weight flavoring agent, such as 10% to 40% by weight flavoring agent, such as 15% to 35% by weight flavoring agent and including 15% to 25% by weight flavoring agent.

In some embodiments, the solid oral dosage composition may further include one or more binders. By binder is meant a composition used in the formulation to hold the active pharmaceutical ingredient (i.e., ibuprofen) and inactive ingredients together in a cohesive mix. Binders in solid oral dosage compositions of the invention may include but are not limited to starches, cellulose ethers, polyvinyl pyrrolidone, silicified microcrystalline cellulose, pregelatinized maize starch, among other binders. For example, the binder in ibuprofen solid oral dosage compositions of interest may be silicified microcrystalline cellulose. In embodiments of the invention, subject ibuprofen solid oral dosage compositions are formulated to be administered in an oral cavity of a subject and maintained for 15 minutes or more to deliver ibuprofen to the subject. Depending on whether the solid oral dosage compositions of interest are maintained in the oral cavity for 15 minutes or more to dissolve, disintegrate by mastication or orally disintegrate without mastication, the amount of binder employed to hold the composition together as a solid cohesive composition may vary. For example, where the ibuprofen solid oral dosage composition is formulated to be maintained in the oral cavity for 15 minutes or more to dissolve the composition, a larger amount of binder may be incorporated into the subject solid oral dosage composition (e.g., an amount required to keep the solid oral dosage composition as a cohesive solid for the entire time the composition is maintained in the oral cavity). In other embodiments, where the solid oral dosage composition is formulated to be orally disintegrated without mastication (e.g., orally disintegratable tablets), only a smaller amount of binder may be incorporated into the subject solid oral dosage composition (e.g., the minimal amount of binder required to keep the solid oral dosage composition together as a cohesive solid until administering the composition in the oral cavity of the subject). As such, the amount of binder present in solid oral dosage compositions of the invention may vary, depending on the amount of ibuprofen, as described above as well as the size and administration type of the composition desired. In some embodiments, the amount of binder in solid oral dosage compositions of interest may be 5 mg or greater, such as 10 mg or greater, such as 25 mg or greater, such as 50 mg or greater, such as 100 mg or greater, such as 200 mg or greater, such as 400 mg or greater. As such, the percent by weight of binder in solid oral dosage compositions of interest may vary, ranging from 0.1 % to 75% by weight binder, such as 1 % to 70% by weight binder, such as 5% to 50% by weight binder, such as 10% to 40% by weight binder, such as 15% to 35% by weight binder and including 15% to 25% by weight binder. In certain embodiments, no binder is incorporated into the ibuprofen solid oral dosage composition.

As described in greater detail below, certain embodiments of the subject ibuprofen solid oral dosage compositions are formulated to be administered in an oral cavity of a subject and maintained for 15 minutes to deliver ibuprofen to the subject. In some embodiments, solid oral dosage compositions of interest are administered in an oral cavity of a subject and maintained in the oral cavity for 15 minutes to dissolve the solid oral dosage composition. In other embodiments, the subject solid oral dosage compositions are administered in an oral cavity of a subject and maintained in the oral cavity for an amount of time sufficient to disintegrate the solid oral dosage composition by mastication (e.g., chewable tablets) or to orally disintegrate the solid oral dosage composition without mastication (e.g., orally disintegrating tablets). The amount of time that ibuprofen solid oral dosage compositions of interest are maintained within the oral cavity may vary depending on the technique of administration (e.g., dissolution, mastication, etc.), type of ailment, size of the solid oral dosage composition, dosage amount, physiology of the subject and position within the oral cavity. In some embodiments, ibuprofen solid oral dosage compositions are formulated to be maintained in the oral cavity for 15 minutes or more and including 20 minutes or more. For example, where the subject ibuprofen-methacrylic acid copolymer solid oral dosage compositions are formulated for dissolution in the oral cavity, the composition may be formulated to dissolve in the oral cavity in 15 minutes or more and including 20 minutes or more.

The amount of ibuprofen released by the solid oral dosage composition during administration may vary. For example, 5 mg or more of ibuprofen may be released during administration, such as 10 mg or more, such as 20 mg or more, such as 40 mg or more, such as 100 mg or more, such as 150 mg or more, such as 200 mg or more, and including 400 mg or more of ibuprofen may be released during administration. As such, 5% or more of the total amount ibuprofen contained in the solid oral dosage composition may be released during administration, such as 10% or more, such as 25% or more, such as 50% or more, such as 75% or more, such as 90% or more, such as 95% or more, and including 99% or more of the total amount ibuprofen contained in the composition may be released during administration. In certain instances, 100% of the ibuprofen is released into the oral cavity during administration.

In certain embodiments, ibuprofen solid oral dosage compositions of the present invention may also provide a sustained release composition of ibuprofen to the subject. As described above, by "sustained release" is meant that the composition is formulated to provide for continuous delivery of ibuprofen over the entire time the solid oral dosage composition is maintained in the oral cavity, such as 15 minutes or longer, such as 30 minutes or longer, and including 60 minutes or longer. The amount of ibuprofen released by the solid oral dosage composition during different phases of dissolution may vary depending on the desired therapeutic effect. For example, in certain instances an immediate therapeutic effect (i.e., acute reduction in pain) is desired. In these instances, the ibuprofen solid oral dosage compositions may be formulated to release a large amount of the ibuprofen immediately upon contact in the oral cavity, such as 50% or more, such as 60% or more, such as 70% or more and including 90% or more of the ibuprofen is released immediately upon contact with the oral cavity.

In other instances, a constant therapeutic effect may be desired. In these instances, the ibuprofen solid oral dosage compositions may be formulated to release ibuprofen at a steady state. For example, in a constant release assay, solid oral dosage compositions of interest may be dissolved in an assay solution (such as saliva or simulated salivary fluid, as described in the experimental section below) to release ibuprofen at a constant rate during dissolution. At predetermined time points, the amount of ibuprofen released may be determined by assessing the amount of ibuprofen remaining in the solid oral dosage composition or by assessing the amount of ibuprofen present in the assay solution. The rate of ibuprofen release during dissolution is given by dividing the mass of ibuprofen by assay time. Where ibuprofen is released at a steady state, ibuprofen solid oral dosage compositions of the present invention will release ibuprofen at a constant rate, such as 1 mg per minute or more, such as 2 mg per minute or more, such as 5 mg per minute or more, such as 10 mg per minute or more, such as 20 mg per minute or more and including 25 mg per minute or more. The amount of ibuprofen present in the assay solution or amount of ibuprofen remaining in the solid oral dosage composition may be assessed using any convenient protocol, such as for example, mass spectrometry, flame ionization spectrometry, high-performance liquid chromatography, among other protocols.

As described above, the subject solid oral dosage compositions include ibuprofen and a methacrylic acid copolymer in an amount sufficient to make the composition organoleptically acceptable for administering (e.g., dissolution, mastication) in an oral cavity of a subject. In embodiments of the invention, the ibuprofen solid oral dosage compositions do not include a cyclodextrin as a taste masking agent. As such, an aspect of certain embodiments is that the bitter and unpleasant taste of ibuprofen in the subject solid oral dosage compositions is masked in the absence of cyclodextrin. The term cyclodextrin is used in its conventional sense to refer to the family of macrocyclic sugar compounds, where sugar monomers are covalently bonded together to form one or more macrocycles. Accordingly, ibuprofen solid oral dosage compositions as described herein do not contain any amount of cyclodextrin, such as α-, β- or γ-cyclodextrins.

In certain embodiments, the present invention provides ibuprofen solid oral dosage compositions which consist of ibuprofen, a methacrylic acid copolymer in an amount sufficient to make the solid oral dosage composition organoleptically acceptable for adminstering (e.g., dissolution, mastication) in an oral cavity of a subject and a buffer. In some instances, the present invention provides ibuprofen solid oral dosage compositions which consist of ibuprofen, a methacrylic acid copolymer in an amount sufficient to make the solid oral dosage composition organoleptically acceptable for dissolution in an oral cavity of a subject, a diluent, a lubricant, one or more souring agents, a flavoring agent, a binder, one or more sweeteners and a buffer. The claimed present invention provides ibuprofen solid oral dosage compositions which consist of ibuprofen, a methacrylic acid copolymer in an amount sufficient to make the solid oral dosage composition organoleptically acceptable for disintegration (e.g., mastication) in an oral cavity of a subject, a diluent, a disintegrant, a lubricant, one or more souring agents, a flavoring agent, a binder, one or more sweeteners and a buffer.

Ibuprofen solid oral dosage compositions of the invention may further include one or more pharmaceutically acceptable excipients as part of a pharmaceutical composition. Excipients may include, but are not limited to, inorganic salts, antimicrobial agents, antioxidants, surfactants, water, alcohols, polyols, glycerine, vegetable oils, phospholipids, and any combinations thereof. Inorganic salts may include, but are not limited to citrate, sodium chloride, potassium chloride, sodium sulfate, sodium phosphate monobasic, sodium phosphate dibasic, and any combinations thereof.

In certain embodiments, ibuprofen solid oral dosage compositions of the invention may also include an antimicrobial agent for preventing or deterring microbial growth, such as for example benzalkonium chloride, benzethonium chloride, benzyl alcohol, cetylpyridinium chloride, chlorobutanol, phenol, phenylethyl alcohol, thimersol, and any combinations thereof.

One or more antioxidants may also be employed. Antioxidants, which can reduce or prevent oxidation and thus deterioration of the ibuprofen solid oral dosage composition, may include, for example, ascorbyl palmitate, butylated hydroxyanisole, butylated hydroxytoluene, monothioglycerol, propyl gallate, sodium bisulfite, sodium formaldehyde sulfoxylate, sodium metabisulfite, and any combinations thereof.

One or more surfactants may also be included in compositions of the invention. For example, suitable surfactants may include, but are not limited to polysorbates, such as "Tween 20" and "Tween 80," and pluronics such as F68 and F88 (BASF, Mount Olive, New Jersey); sorbitan esters; lipids, such as phospholipids such as lecithin and other phosphatidylcholines, phosphatidylethanolamines (although preferably not in liposomal form), fatty acids and fatty esters; steroids, such as cholesterol; chelating agents, such as EDTA.

The presence of any individual excipient in the ibuprofen solid oral dosage composition will vary depending on the nature and function of the excipient and particular needs of the composition in different solid oral dosage forms. Typically, the optimal amount of any individual excipient is determined through routine experimentation, i.e., by preparing compositions containing varying amounts of the excipient (ranging from low to high), examining the stability, irritant taste-masking efficacy and other parameters, and then determining the range at which optimal performance is attained with no significant adverse effects.

### METHODS FOR PREPARING AN ORGANOLEPTICALY ACCEPTABLE SOLID ORAL DOSAGE COMPOSITION OF IBUPROFEN

As summarized above, ibuprofen solid oral dosage claimed compositions of interest are organoleptically acceptable compositions having ibuprofen and a methacrylic acid copolymer formulated for administering (e.g., dissolution, mastication) in an oral cavity of a subject. Aspects of the invention also include methods for preparing the subject organoleptically acceptable ibuprofen solid oral dosage compositions. In certain embodiments, methods for preparing the claimed ibuprofen solid oral dosage compositions may be characterized by a first process of producing an intermediate ibuprofen-methacrylic acid copolymer granulate, which includes the active agent (i.e., ibuprofen) and a methacrylic acid copolymer, and then a second process of producing the final organoleptically acceptable solid oral dosage composition from the intermediate ibuprofen granulate. In certain embodiments, methods for preparing ibuprofen solid oral dosage compositions of interest include mixing ibuprofen with a methacrylic acid copolymer and a buffer to produce a ibuprofen-methacrylic acid copolymer composition; granulating the ibuprofen-methacrylic acid copolymer composition with water to produce a wet ibuprofen-methacrylic acid copolymer granulate; drying the wet ibuprofen-methacrylic acid copolymer granulate; milling the dried ibuprofen-methacrylic acid copolymer granulate to produce an intermediate ibuprofen-methacrylic acid copolymer granulate composition; mixing the intermediate ibuprofen-methacrylic acid copolymer granulate composition with a diluent, a disintegrant, a binder, a lubricant, a souring agent, a sweetener and a flavoring agent; and tableting the mixture to produce an organoleptically acceptable ibuprofen-methacrylic acid copolymer solid oral dosage composition as claimed. In yet other embodiments, methods for preparing the claimed ibuprofen solid oral dosage compositions include mixing ibuprofen with a methacrylic acid copolymer and a buffer to produce an ibuprofen-methacrylic acid copolymer composition and granulating (e.g., with water) the ibuprofen-methacrylic acid copolymer composition to produce an ibuprofen-methacrylic acid copolymer granulate which includes a matrix having methacrylic acid copolymer-ibuprofen polyelectrolyte complexes. In these embodiments, the polyelectrolyte complexes of methacrylic acid copolymer-ibuprofen may be formed during the granulation process and are resistant to shearing or disintegrating forces during granulation, drying, compression tableting. In certain instances, methods include granulating (e.g., with water) the ibuprofen-methacrylic acid copolymer composition to produce an ibuprofen-methacrylic acid copolymer granulate having a matrix which includes methacrylic acid copolymer-ibuprofen polyelectrolyte complexes as nanoparticles.

In methods of the claimed invention, an amount of ibuprofen, one or more methacrylic acid copolymers and a buffer are mixed to produce an ibuprofen-methacrylic acid copolymer composition. The ibuprofen-methacrylic acid copolymer composition may be mixed by any convenient mixing protocol, such as but not limited to planetary mixers, Patterson-Kelley blender, hand mixers, standup mixers, inline mixers, powder liquid mixers, batch mixers, kneaders, agitator drives, impellers, hydrofoil mixers, aerators, among other mixing protocols.

In some embodiments, all of the components of the ibuprofen-methacrylic acid copolymer composition (i.e., ibuprofen, methacrylic acid copolymer and buffer) are added to the mixer simultaneously. In other embodiments, each component may be added to the mixer sequentially. In certain embodiments, each component may be added to the mixer in a specific order. For example, in certain instances, methods include adding the components of the ibuprofen-methacrylic acid composition to the mixer in the order: 1) a half amount of the methacrylic acid copolymer; 2) the buffer; 3) the ibuprofen and 4) the remaining half amount of methacrylic acid copolymer. In some embodiments, one or more components may be mixed concurrently while being added to the mixer. In other embodiments, all of the components are first added to the mixer and then the entire composition is mixed.

In some embodiments, the particle size of each of the ibuprofen, the claimed methacrylic acid copolymer and buffer may be reduced before mixing the components together. The particle size may be reduced by any convenient protocol and may include but is not limited to lump breakers, hammermills, fine grinders, classifier mills or sifters, among other particle size reduction protocols. In certain embodiments, to reduce the particle size, each component is passed through a mesh screen. Depending on the particle size desired, the mesh screen may vary. In some embodiments, the mesh screen is a 2 mesh screen or smaller, such as a 4 mesh screen or smaller, such as a 10 mesh screen or smaller, such as a 20 mesh screen or smaller, such as a 30 mesh screen or smaller, such as a 40 mesh screen or smaller, and including a 60 mesh screen or smaller. For example, each component may be passed through a 20 mesh screen before adding them to the mixer. In certain embodiments, methods include passing the components of the ibuprofen-methacrylic acid copolymer composition through a 20 mesh screen in the order: 1) a half amount of the methacrylic acid copolymer; 2) the buffer; 3) the ibuprofen and 4) the remaining half amount of methacrylic acid copolymer.

In embodiments of the invention, the ibuprofen-methacrylic acid copolymer composition is mixed for an amount of time sufficient to incorporate each component and to produce a homogenous mixture. For example, the ibuprofen-methacrylic acid copolymer may be mixed for 1 minute or more, such as 2 minutes or more, such as 3 minutes or more, such as 5 minutes or more, such as 10 minutes or more, and including 15 minutes or more.

After the ibuprofen-methacrylic acid copolymer composition is sufficiently mixed, the ibuprofen-methacrylic acid copolymer composition is granulated. The term granulate is used in its conventional sense to refer to the process of forming the ibuprofen-methacrylic acid copolymer composition into granules. In other words, the powder particles of the ibuprofen-methacrylic acid copolymer composition are agglomerated or made to adhere to each other to form, larger multiparticle entities such as granules and may also include pelletizing or spheronisation. The ibuprofen-methacrylic acid copolymer composition may be granulated by either wet or dry granulation.

In certain embodiments, the ibuprofen-methacrylic acid copolymer composition is granulated into ibuprofen-methacrylic acid copolymer granules by wet granulation. In these embodiments, pH-adjusted water is added to the ibuprofen-methacrylic acid copolymer composition to granulate the composition into a wet ibuprofen-methacrylic acid copolymer granulate. During wet granulation, the pH adjusted water may be added to the ibuprofen-methacrylic acid copolymer composition at a rate that varies, ranging from 10 to 50 g per minute, such as 10 to 40 g per minute, such as 10 to 30 g per minute and including 15 to 20 g per minute. By pH adjusted water is meant the pH of the water added to granulate the ibuprofen-methacrylic acid copolymer composition is either increased or decreased, as desired. In some embodiments, the pH of the water is adjusted to have a pH which ranges from 7 to 8, such as 7.1 to 7.9, such as 7.2 to 7.8 and including a pH of from 7.3 to 7.7. In certain embodiments, the pH of the water added to granulate the ibuprofen-methacrylic acid copolymer composition is adjusted to have a pH which ranges from 7.3 to 7.5. The pH of the water may be adjusted using any convenient protocol. In some embodiments, the pH is decreased by adding an acid (e.g., HCl). In other embodiments, the pH is increased by adding a base (e.g., NaOH). In certain embodiments, the pH of the water may be adjusted by adding a buffer to the water, such as by adding an amount of sodium citrate dihydrate.

The rate of granulation may vary, ranging from 10 to 50 g per minute, such as 10 to 40 g per minute, such as 10 to 30 g per minute and including 15 to 25 g per minute. In certain embodiments, the ibuprofen-methacrylic acid copolymer composition is granulated at a rate of 15 to 20 g per minute.

In some embodiments, after wet granulation of the ibuprofen-methacrylic acid copolymer composition, the wet granulate is passed through a mesh screen. Depending on the particle size of the granulate desired, the mesh screen may vary. In some embodiments, the mesh screen is a 2 mesh screen or smaller, such as a 4 mesh screen or smaller, such as a 10 mesh screen or smaller, such as a 20 mesh screen or smaller, such as a 30 mesh screen or smaller, such as a 40 mesh screen or smaller, and including a 60 mesh screen or smaller. In certain embodiments, the ibuprofen-methacrylic acid copolymer granulation is passed through a 10 mesh screen after granulation.

Where the ibuprofen-methacrylic acid copolymer composition is wet granulated, methods further include drying the wet ibuprofen-methacrylic acid copolymer granulate. The wet granulate may be dried using any convenient protocol. For example, drying may also be achieved by spray drying the wet granulate, where water is removed by a gaseous stream at an elevated temperature. In certain instances, the wet ibuprofen-methacrylic acid copolymer granulate is air-dried by blowing air (e.g., room temperature or heated air) over the wet granulate. In these instances, the temperature employed during air-drying may vary, so long as the temperature is sufficient to dry the wet granulation without altering or damaging the granules. For instance, the temperature may range from 40 °C to 70 °C, such as 45 °C to 65 °C, such as 50 °C to 60 °C and including 50 °C to 55 °C.

In some embodiments, the wet granulate is air-dried until the granulate reaches a desired consistency, such as where the granulate contains 5% water or less, such as 4% water or less, such as 3% water or less and including until the granulation contains 2% water or less. In other embodiments, the wet granulate is air-dried until the granulate reaches a predetermined threshold in a loss on drying (LOD) analysis. For example, the wet granulate may be air-dried until the granulate reaches a 5% LOD or less, such as 4% LOD or less, such as a 3% LOD or less and including 2% LOD or less.

After drying the ibuprofen-methacrylic acid copolymer granulate, the dried ibuprofen-methacrylic acid copolymer granulation is milled to produce an intermediate ibuprofen-methacrylic acid copolymer granulate composition. By milling is meant, grinding or otherwise processing the dried ibuprofen-methacrylic acid copolymer granulate to reduce the particle size of the dried granulate. The granulate may be milled by any convenient milling protocol, for example, round impellers, axial flow impellers, radial flow impellers, ball mill, rod mill, autogenous mill, pebble mill, grinding rolls, buhrstone mills, semi-autogenous mill, vibratory mill or roller mill, among other protocols. Depending on the desired density and size of granules, the dried ibuprofen-methacrylic acid copolymer granulate may be milled at any suitable rate as desired .

In some embodiments, the ibuprofen-methacrylic acid copolymer granulate is concurrently milled through a mesh screen. Depending on the particle size of the granules desired, the mesh screen may be a 2 mesh screen or smaller, such as a 4 mesh screen or smaller, such as a 10 mesh screen or smaller, such as a 20 mesh screen or smaller, such as a 30 mesh screen or smaller, such as a 40 mesh screen or smaller, and including a 60 mesh screen or smaller. In certain instances, the ibuprofen-methacrylic acid copolymer granulate is concurrently milled through a 20 mesh screen. In other embodiments, the ibuprofen-methacrylic acid copolymer granulate is passed through a mesh screen after the granulate has been milled.

In some embodiments, the intermediate ibuprofen-methacrylic acid granulate composition (i.e., the milled ibuprofen-methacrylic acid copolymer granulate) is mixed with one or more of a diluent, disintegrant, binder, lubricant, souring agent, sweetener and a flavoring agent to produce an ibuprofen-methacrylic acid copolymer solid oral dosage composition precursor. The intermediate ibuprofen-methacrylic acid copolymer granulate composition may be mixed with one or more of a diluent, disintegrant, binder, lubricant, souring agent, sweetener and a flavoring agent by any convenient mixing protocol, such as but not limited to planetary mixers, Patterson-Kelley blender, hand mixers, standup mixers, inline mixers, powder liquid mixers, batch mixers, kneaders, agitator drives, impellers, hydrofoil mixers, aerators, among other mixing protocols.

In some embodiments, all of the components (i.e., intermediate ibuprofen composition and one or more of a diluent, disintegrant, binder, lubricant, souring agent, sweetener and a flavoring agent) are added to the mixer simultaneously. In other embodiments, each component is added to the mixer sequentially.

In certain embodiments, each component may be added to the mixer in a specific order. For example, in certain instances, methods include adding the components to the mixer in the order: 1) a half amount of a diluent; 2) the intermediate ibuprofen composition (i.e., the milled ibuprofen-methacrylic acid copolymer granulate); 3) a flavoring agent; 4) a sweetener; 5) a souring agent; 6) a binder; and 7) the remaining half amount of diluent. In other instances, methods include adding the components to the mixer in the order: 1) a half amount of a diluent; 2) the intermediate ibuprofen composition (i.e., the milled ibuprofen-methacrylic acid copolymer granulate); 3) a flavoring agent; 4) a sweetener; 5) a souring agent; 6) a binder; 7) a disintegrant; and 8) the remaining half amount of diluent. Where the components are added sequentially, the components may be mixed concurrently while being added to the mixer. In other embodiments, all of the components are first added to the mixer and then the entire composition is mixed.

In some embodiments, before adding to the mixer, each component is passed through a mesh screen. The mesh screen may be any convenient mesh screen sufficient to reduce the granular size of each component, as desired. In some embodiments, the mesh screen is a 2 mesh screen or smaller, such as a 4 mesh screen or smaller, such as a 10 mesh screen or smaller, such as a 20 mesh screen or smaller, such as a 30 mesh screen or smaller, such as a 40 mesh screen or smaller, and including a 60 mesh screen or smaller. For example, each component may be passed through a 20 mesh screen before adding to the mixer. In certain embodiments, methods include passing the components through a 20 mesh screen in the order: 1) a half amount of a diluent; 2) the intermediate ibuprofen composition (i.e., the milled ibuprofen-methacrylic acid copolymer granulate); 3) a flavoring agent; 4) a sweetener; 5) a souring agent; 6) a binder; and 7) the remaining half amount of diluent. In other embodiments, methods include passing the components through a 20 mesh screen in the order: 1) a half amount of a diluent; 2) the intermediate ibuprofen composition (i.e., the milled ibuprofen-methacrylic acid copolymer granulate); 3) a flavoring agent; 4) a sweetener; 5) a souring agent; 6) a binder; 7) a disintegrant and 8) the remaining half amount of diluent.

In embodiments of the invention, the ibuprofen-methacrylic acid copolymer solid oral dosage composition precursor is mixed for an amount of time sufficient to incorporate each component and to produce a homogenous mixture. In some instances, the ibuprofen-methacrylic acid copolymer solid oral dosage composition precursor is mixed for 1 minute or more, such as 2 minutes or more, such as 3 minutes or more, such as 5 minutes or more, and including 10 minutes or more.

In certain embodiments, a lubricant may be added to the ibuprofen-methacrylic acid copolymer solid oral dosage composition precursor. In some instances, the lubricant is added to the ibuprofen-methacrylic acid copolymer solid oral dosage composition precursor after mixing the components as described above. In other instances, the lubricant is added concurrently while mixing the components as described above. For example, in certain instances a lubricant is passed through a screen mesh (e.g., a 40-mesh screen) into the mixing ibuprofen-methacrylic acid copolymer solid oral dosage composition precursor and mixed for an additional amount of time, such as 5 minutes or more, such as 10 minutes or more and including 15 minutes or more.

After incorporating all of the desired components, the subject organoleptically acceptable ibuprofen-methacrylic acid copolymer solid oral dosage claimed compositions may be produced from the ibuprofen-methacrylic acid copolymer solid oral dosage composition precursor by any convenient powder compression protocol, such as by tableting, pelletization, among others. In some embodiments, solid oral dosage compositions of interested are produced by tableting the ibuprofen-methacrylic acid copolymer solid oral dosage composition precursor in a manner sufficient to produce a tablet having a hardness ranging from 2 to 25 kP (kilopond) per cm², such as 3 to 22 kP per cm², such as 5 to 20 kP per cm², such as 5 to 15 kP per cm², such as 5 to 12 kP per cm² and including such as 5 to 10 kP per cm². The hardness of tableted ibuprofen solid oral dosage compositions may be determined using any convenient protocol, including but not limited to a Monsanto hardness tester, Strong-Cobb hardness tester, VarianVK hardness tester, Pfizer hardness tester, Erwecka hardness tester or Schleuniger hardness tester, among other hardness testers. Where ibuprofen-methacrylic acid copolymer solid oral dosage compositions are formulated as a chewable tablets, the composition may be tableted to have a hardness that is facily fragmented and broken down by chewing or grinding. Depending on the amount of disintegrant present, size of the tablet and physiology of the subject (e.g. child or person with unhealthy teeth), ibuprofen-methacrylic acid copolymer solid oral dosage compositions formulated as cheweable tablets have a hardness that readily fragments during mastication and is safe such that the subject experiences no adverse effects to the teeth or gums during mastication.

Likewise, where ibuprofen-methacrylic acid copolymer solid oral dosage claimed compositions are formulated as a orally disintegrating tablets, the composition may be tableted to have a hardness that is facily fragmented and broken down in the presence of little or no chewing. Depending on the amount of disintegrant present, size of the tablet and physiology of the subject, ibuprofen-methacrylic acid copolymer solid oral dosage compositions formulated as orally disintegrating tablets have a hardness that readily fragments and breaks down when placed in the oral cavity.

The subject organoleptically acceptable ibuprofen solid oral dosage compositions may be tableted into any size as desired, for example ranging from 100 to 1000 mg tablets such as 250 to 950 mg tablets, such as 300 to 900 mg tablets, such as 350 to 850 mg tablets, such as 400 to 800 mg tablets, such as 450 to 750 mg tablets and including tablets ranging from 760 to 840 mg.

The properties of the ibuprofen-methacrylic acid copolymer composition, wet ibuprofen-methacrylic acid copolymer granulate, intermediate ibuprofen-methacrylic acid copolymer granulate composition and ibuprofen-methacrylic acid copolymer solid oral dosage composition precursor may be characterized at any phase during methods of the invention. The term characterizing is used to refer to the analysis of one or more of the properties and/or components of the ibuprofen-methacrylic acid copolymer composition, wet ibuprofen-methacrylic acid copolymer granulate, intermediate ibuprofen-methacrylic acid copolymer granulate composition and ibuprofen-methacrylic acid copolymer solid oral dosage composition precursor. Characterizing may include, but is not limited to, determining the organic composition, bacterial content, pH, physical properties (e.g., granulate density, water content), content assay (API), spectroscopic properties, particle size distribution and impurity composition (trace metals, relating substances, etc.),. Methods for analyzing compositions of the invention may include, but are not limited to the use of high performance liquid chromatography (HPLC) and moisture analyzers .

In some embodiments, methods include monitoring each of the ibuprofen-methacrylic acid copolymer composition, wet ibuprofen-methacrylic acid copolymer granulate, intermediate ibuprofen-methacrylic acid copolymer granulate composition and ibuprofen-methacrylic acid copolymer solid oral dosage composition precursor throughout the entire method for preparing the subject organoleptically acceptable ibuprofen solid oral dosage compositions. In some embodiments, monitoring includes collecting real-time data (e.g., pH, content assay, moisture content), such as by employing a detector to monitor each composition. In other embodiments, monitoring includes characterizing each composition at regular intervals, such as every 1 minute, every 5 minutes, every 10 minutes, every 30 minutes, every 60 minutes or some other interval. In yet other embodiments, methods include characterizing each composition as each step is completed.

In some embodiments, methods of the invention also include assessing the properties of the characterized composition. By "assessing" is meant that a human (either alone or with the assistance of a computer, if using a computer-automated process initially set up under human direction), evaluates the determined composition and determines whether the composition is suitable or unsuitable to continue on to the next step of processing. If after assessing that the determined composition is suitable, each composition may proceed to the following step without any further adjustments. In other words, methods of these embodiments include a step of assessing the determined composition to identify any desired adjustments. For example, in certain embodiments, it may be desired to further reduce the particle size of the intermediate ibuprofen-methacrylic acid copolymer granulate composition before adding one or more of the diluent, disintegrant binder, lubricant, souring agent, sweetener and a flavoring agent. In other embodiments, it may be desired to produce a more homogenous wet granulation before drying.

Aspects of the invention also include organoleptically acceptable ibuprofen solid oral dosage claimed compositions produced by the process of preparing an organoleptically acceptable ibuprofen solid oral dosage compositions as described in detail above. For example, solid oral dosage compositions of interest may include organoleptically acceptable ibuprofen solid oral dosage compositions produced by the method of first producing an intermediate ibuprofen-methacrylic acid copolymer granulate composition, followed by incorporating pharmaceutically acceptable excipients with the intermediate ibuprofen-methacrylic acid copolymer granulate composition.

In certain embodiments, organoleptically acceptable ibuprofen solid oral dosage compositions of interest include compositions produced by the method of mixing ibuprofen with a methacrylic acid copolymer and a buffer to produce a ibuprofen-methacrylic acid copolymer composition; granulating the ibuprofen-methacrylic acid copolymer composition with water to produce a wet ibuprofen-methacrylic acid copolymer granulate; drying the wet ibuprofen-methacrylic acid copolymer granulate; milling the dried ibuprofen-methacrylic acid copolymer granulate to produce an intermediate ibuprofen-methacrylic acid copolymer granulate composition; mixing the ibuprofen-methacrylic acid copolymer granulate composition with one or more of a diluent, a disintegrant, a binder, a lubricant, a souring agent, a sweetener and a flavoring agent to produce an ibuprofen-methacrylic acid copolymer solid oral dosage composition precursor and tableting the ibuprofen-methacrylic acid copolymer solid oral dosage composition precursor to produce an organoleptically acceptable ibuprofen-methacrylic acid copolymer solid oral dosage composition.

In some embodiments, organoleptically acceptable ibuprofen solid oral dosage compositions of interest may include solid oral dosage compositions where the ibuprofen-methacrylic acid copolymer composition is produced by mixing in the order: 1) a half amount of the methacrylic acid copolymer; 2) the buffer; 3) the ibuprofen and 4) the remaining half amount of methacrylic acid copolymer. In other embodiments, organoleptically acceptable ibuprofen solid oral dosage compositions of interest may include compositions where ibuprofen-methacrylic acid copolymer granulate is produce by wet granulation using pH-adjusted water. In other embodiments, organoleptically acceptable ibuprofen solid oral dosage compositions of interest may include compositions where an intermediate ibuprofen-methacrylic acid copolymer granulate composition is produced by milling the dried ibuprofen-methacrylic acid copolymer granulate. In yet other embodiments, organoleptically acceptable ibuprofen solid oral dosage compositions of interest may include compositions where the ibuprofen-methacrylic acid copolymer solid oral dosage composition precursor is produced by blending the intermediate ibuprofen granulate composition with one or more pharmaceutically acceptable excipients. In these embodiments, organoleptically acceptable ibuprofen solid oral dosage compositions of interest may include compositions where the pharmaceutically acceptable excipients are blended with the intermediate ibuprofen-methacrylic acid copolymer granulate composition in the order: 1) a diluent; 2) intermediate ibuprofen-methacrylic acid copolymer granulate; 3) flavoring agent; 4) sweetener; 5) souring agent; 6) binder and 7) lubricant. In other embodiments, organoleptically acceptable ibuprofen solid oral dosage compositions of interest may include compositions where the pharmaceutically acceptable excipients are blended with the intermediate ibuprofen-methacrylic acid copolymer granulate composition in the order: 1) a diluent; 2) intermediate ibuprofen-methacrylic acid copolymer granulate; 3) flavoring agent; 4) sweetener; 5) souring agent; 6) binder; 7) disintegrant and 8) lubricant.

### METHODS FOR USING AN ORGANOLEPTICALY ACCEPTABLE SOLID ORAL DOSAGE claimed COMPOSITION OF IBUPROFEN

As summarized above, aspects of the invention also include methods for treating oral-esophageal pain by administering an ibuprofen solid oral dosage composition to a subject. The term oral-esophageal is used in its conventional sense to refer the anatomical structures of the mouth and throat, including the nasal cavity. As such, oral-esophageal pain may include pain or inflammation originating in the buccal cavity, the sublingual cavity, the nasal cavity, upper palate, lower palate, gums, lips, jaw, tongue, esophagus, larynx, pharynx, epiglottis, tonsils, salivary glands or lymph nodes of the neck. In certain embodiments, methods of the present invention include treating a subject having pain in the laryngopharynx (i.e., sore throat).

In describing methods of the present invention, the term "subject" is meant the person or organism to which the ibuprofen-methacrylic acid copolymer solid oral dosage composition is applied and maintained in contact. As such, subjects of the invention may include but are not limited to mammals, e.g., humans and other primates, such as chimpanzees and other apes and monkey species; and the like, where in certain embodiments the subject are humans. The subject may be one that has been diagnosed as having an oral-esophageal pain condition (e.g., sore throat), where the subject may have been one that has been diagnosed by a health care professional as having the condition.

In some embodiments, the subject ibuprofen-methacrylic acid copolymer solid oral dosage claimed compositions are administered to a subject to treat oral-esophageal pain. By treating oral-esophageal pain is meant that administering the ibuprofen-methacrylic acid copolymer solid oral dosage composition to a subject may reduce the amount of oral-esophageal pain reported by the subjectas compared to the reported pain when untreated. In certain embodiments treating oral-esophageal pain includes completely inhibiting, terminating pain such that the subject no longer reports any discomfort from oral-esophageal pain. As such, treating oral-esophageal pain as described herein, includes both curing and managing pain. In certain embodiments, oral-esophageal pain is measured on a pain rating scale of 1-5, where an oral-esophageal pain rating of 1 is the absence of pain and an oral-esophageal pain rating of 5 is unbearable pain. In these embodiments, a pain rating of 1 is the complete absence of pain, a pain rating of 2 is minor to slight pain, a pain rating of 3 is moderate pain, a pain rating of 4 is severe pain and a pain rating of 5 is unbearable pain. Where the ibuprofen-methacrylic acid copolymer solid oral dosage composition of the invention are administered to a subject, oral-esophageal pain may be reduced by 1 pain rating or more, such as 2 pain ratings or more, such as 3 pain ratings or more and including 4 pain ratings or more. For example, administering the ibuprofen-methacrylic acid copolymer solid oral dosage composition of the invention may reduce oral-esophageal pain from a pain rating of 5 to a pain rating of 4 or less, such as a pain rating of 3 or less, such as a pain rating of 2 or less, and including reducing oral-esophageal pain from a pain rating of 5 to a pain rating of 1.

Oral-esophageal pain may be determined by any convenient protocol, such as for example, by the diagnosis by a health care professional or by self-reporting by the subject.

In practicing methods of the present invention, one or more solid oral dosage compositions having ibuprofen and a methacrylic acid copolymer (as described in detail above) is placed (either by the subject itself or by a caregiver) into an oral cavity of the subject and maintained in the oral cavity of the subject for an amount of time sufficient to treat the subject for the oral-esophageal pain. By oral cavity is meant a region within the mouth of the subject and may include but is not limited to, the buccal cavity, sublingual cavity, the upper palate, along the gums, near the throat and on top of the tongue of the subject.

Depending on the type of ailment, physiology of the subject, and type of solid oral dosage composition, the delivery of ibuprofen to the subject may be local or systemic. In certain embodiments, methods include locally treating oral-esophageal pain. The term "local" is used herein in its conventional sense to refer to directly delivering ibuprofen to a location which is at or near the site of administration of the solid oral dosage composition. For example, in certain instances, methods include placing one or more of the subject solid oral dosage compositions into an oral cavity of the subject and maintaining the solid oral dosage composition in the oral cavity for an amount of time sufficient to dissolve the composition and locally deliver ibuprofen to the location of oral-esophageal pain. As discussed above, depending on the type of solid oral dosage composition and therapeutic effect desired, local delivery of ibuprofen may be sustained local delivery or incremental local delivery. For example, local delivery of ibuprofen may be constant and continuous such that delivery of ibuprofen occurs over the entire time the solid oral dosage composition is maintained in the oral cavity, such as over the course of 5 minutes or more, such as 10 minutes or more, such as 15 minutes or more, and including 20 minutes or more. In other instances, local delivery of ibuprofen may be delivered incrementally such that ibuprofen is locally delivered to the location of oral-esophageal pain at discrete times while the solid oral dosage composition is maintained in the oral cavity. For example, where the ibuprofen solid oral dosage composition is maintained in the oral cavity for an amount of time sufficient to dissolve the composition, ibuprofen may be delivered at specific times while the solid oral dosage composition is being dissolved. Depending on the rate of dissolution of the solid oral dosage composition in the oral cavity (e.g., due to the physiology of the subject or method of dissolution such as sucking on the composition), ibuprofen may be locally delivered every 1 minute while the solid oral dosage composition is maintained in the oral cavity, such as every 2 minutes, such as every 5 minutes and including every 10 minutes while the solid oral dosage composition is maintained in the oral cavity. In other embodiments, ibuprofen may be delivered to the oral cavity after certain percentages of the solid oral dosage composition is dissolved. For example, ibuprofen may be delivered after every 10% of the solid oral dosage composition is dissolved in the oral cavity, such as every 15% of the solid oral dosage composition is dissolved, such as every 20% of the solid oral dosage composition is dissolved, such as every 25% of the solid oral dosage composition is dissolved, such as every 30% of the solid oral dosage composition is dissolved and including after every 33% or of the solid oral dosage composition is dissolved in the oral cavity.

In other embodiments, methods include systemically administering ibuprofen to the subject. The term "systemic" is used herein in its conventional sense to refer to intestinal absorption of the ibuprofen followed by systemic blood circulation to deliver the ibuprofen to the subject. For example, methods may include placing one or more of the solid oral dosage compositions of interest into the oral cavity of a subject and disintegrating the solid oral dosage composition (e.g., mastication) resulting in intestinal absorption of the ibuprofen and delivering ibuprofen to the subject by systemic blood circulation.

In embodiments of the invention, methods include maintaining the subject solid oral dosage compositions in an oral cavity of a subject for a predetermined amount of time to deliver ibuprofen to the subject. In some embodiments, solid oral dosage compositions of interest are maintained in the oral cavity for an amount of time sufficient to dissolve the solid oral dosage composition. In other embodiments, the subject solid oral dosage compositions are administered in an oral cavity of a subject and disintegrated in the oral cavity by mastication (e.g., chewable tablets) or by disintegration without mastication (e.g., orally disintegrating tablets). The amount of time that ibuprofen solid oral dosage compositions of interest are maintained within the oral cavity may vary depending on the technique of administration (e.g., dissolution, mastication, etc.), type of ailment, size of the solid oral dosage composition, dosage amount, physiology of the subject and position within the oral cavity. In some embodiments, ibuprofen solid oral dosage compositions are maintained in the oral cavity for 15 minutes or more and including 20 minutes or more. For example, where the subject ibuprofen-methacrylic acid copolymer solid oral dosage compositions are dissolved in the oral cavity, the composition may be maintained in the oral cavity for 15 minutes or more and including 20 minutes or more.

As such, methods of the invention may include dissolution of the composition, chewing or grinding the composition (i.e., mastication) using the teeth or gums, or a combination of dissolution and mastication. In certain embodiments, methods include sucking on the composition to dissolve the composition in the oral cavity of the subject. As described above, ibuprofen-methacrylic acid copolymer solid oral dosage claimed compositions of the invention may include tablets, troches, lozenges, chewable tablets or orally disintegrating tablets. As such, the method of administration in the oral cavity will depend on the particular type of solid oral dosage composition administered to the subject. For example, where a chewable tablet is administered to the subject, the solid oral dosage composition will be disintegrated in the oral cavity by mastication. Where a troche or lozenge is administered to the subject, the solid oral dosage composition will be dissolved in the oral cavity.

All or part of the ibuprofen solid oral dosage composition may be dissolved or disintegrated in the oral cavity. For example, 5% or more of the composition may be dissolved or disintegrated, such as 10% or more, such as 25% or more, such as 50% or more, such as 75% or more, such as 90% or more, such as 95% or more such as 99% or more of the, including the entire solid oral dosage composition.

Depending on the size of the ibuprofen solid oral dosage composition, the physiology of the subject and the desired therapeutic effect, the amount of time that the solid oral dosage composition is maintained in the oral cavity for 15 minutes or longer, such as 20 minutes or longer, such as 30 minutes or longer and including 60 minutes or longer.

In certain embodiments, the ibuprofen solid oral dosage claimed composition is maintained in the oral cavity for an amount of time sufficient to release a predetermined amount of ibuprofen from the solid oral dosage composition. For example, the ibuprofen solid oral dosage composition may be maintained in the oral cavity for an amount of time sufficient to release 5% or more of the ibuprofen, such as 10% or more, such as 25% or more, such as 50% or more, such as 75% or more, such as 90% or more, such as 95% or more, and including maintaining the ibuprofen solid oral dosage composition in the oral cavity for an amount of time sufficient to release 99% or more of the ibuprofen. In certain instances, the ibuprofen solid oral dosage composition is maintained in the oral cavity until 100% of the ibuprofen is released.

In other embodiments, the ibuprofen solid oral dosage claimed composition is maintained in the oral cavity of the subject for an amount of time sufficient to deliver a desired dosage of ibuprofen to the subject. For example, the ibuprofen solid oral dosage composition may be maintained in the oral cavity for an amount of time sufficient to deliver 5 mg or more of ibuprofen to the subject, such as 10 mg or more, such as 20 mg or more, such as 40 mg or more, such as 100 mg or more, such as 150 mg or more, such as 200 mg or more, such as 300 mg or more, and including maintaining the solid oral dosage composition in the oral cavity for an amount of time sufficient to deliver 400 mg or more of ibuprofen to the subject.

In other embodiments, the ibuprofen solid oral dosage claimed composition is maintained within an oral cavity of the subject for an amount of time sufficient to provide a particular therapeutic effect (e.g., reduction of oral-esophageal pain) to the subject by the ibuprofen. For example, the ibuprofen solid oral dosage composition may be maintained in the oral cavity for an amount of time sufficient to reduce oral-esophageal pain as reported by the subject by 1 pain rating or more, such as 2 pain ratings or more, such as 3 pain ratings or more and including 4 pain ratings or more on a pain rating scale as described above. For example, the ibuprofen solid oral dosage composition may be maintained in the oral cavity for an amount of time sufficient to reduce oral-esophageal pain as reported by the subject from a pain rating of 5 to a pain rating of 4 or less, such as a pain rating of 3 or less, such as a pain rating of 2 or less, and including reducing oral-esophageal pain from a pain rating of 5 to a pain rating of 1.

In yet other embodiments, the ibuprofen solid oral dosage composition is maintained in the oral cavity for an amount of time sufficient to dissolve a predetermined amount of the ibuprofen solid oral dosage composition. For example, the ibuprofen solid oral dosage composition may be maintained in the oral cavity for an amount of time sufficient to dissolve 5% or more of the ibuprofen solid oral dosage composition, such as 10% or more, such as 25% or more, such as 50% or more, such as 75% or more, such as 90% or more, such as 95% or more, and including maintaining the ibuprofen solid oral dosage composition in the oral cavity for an amount of time sufficient to dissolve 99% or more of the ibuprofen solid oral dosage composition. In certain instances, the ibuprofen solid oral dosage composition is maintained in the oral cavity for an amount of time sufficient to dissolve 100% of the ibuprofen solid oral dosage composition.

In practicing methods of the invention, protocols for treating oral-esophageal pain in a subject may vary, such as for example by age, weight, severity of the pain, the general health of the subject, as well as the particular concentration of the ibuprofen being administered. In embodiments of the invention, the dosage of ibuprofen delivered by oral administration may vary, in some instances, ranging from 5 mg to 800 mg. As such, depending on the physiology of the subject as well as the desired therapeutic effect, the dosage of ibuprofen provided by methods of the invention may range, from 5 to 800 mg, such as 10 to about 500 mg, such as 20 to 400 mg, such as 25 to 350 mg, such as 30 to 300 mg, such as 40 to 250 mg and including 40 to 200 mg.

Therefore, the dosage of ibuprofen solid oral dosage compositions of interest may vary, ranging from about 0.1 mg/kg to 25 mg/kg per day, such as from 0.1 mg/kg to 20 mg/kg per day, such as 0.1mg/kg to 18 mg/kg per day, such as 0.1 mg/kg to 15 mg/kg per day, such as 0.1 mg/kg to 10 mg/kg per day, and including 0.1 mg/kg to 5 mg/kg per day. In other embodiments, the dosage may range from 0.1 to 6.5 mg/kg four times per day (QID), such as 0.1 to 5 mg/kg QID, such as 0.1 mg/kg to 4 mg/kg QID. In other embodiments, the oral dosage may range from 0.01 mg/kg to 8.5 mg/kg three times per day (TID), such as 0.1 mg/kg to 6 mg/kg TID, such as 0.1 mg/kg to 5 mg/kg TID, and including as 0.1 mg/kg to 4 mg/kg TID. In yet other embodiments, the oral dosage may range from 0.1 mg/kg to 13 mg/kg two times per day (BID), such as 0.1 mg/kg to 12 mg/kg BID, such as 5 mg/kg to 10 mg/kg BID, including 0.1 mg/kg to 8 mg/kg BID. The amount of compound administered will depend on the physiology of the subject, the absorptivity of ibuprofen by the subject, as well as the magnitude of therapeutic effect desired. Dosing schedules may include, but is not limited to administration five times per day, four times per day, three times per day, twice per day, once per day, three times per week, twice per week, once per week, twice per month, once per month, and any combination thereof.

In some embodiments, the oral-esophageal pain may be a chronic condition (e.g., cancer) requiring the subject methods and compositions in multiple doses over an extended period. Alternatively, methods and compositions of the invention may be administered to treat an acute condition (e.g., sore throat from a cold or tonsilitis) in single or multiple doses for a relatively short period, for example one to two weeks.

In practicing embodiments of the invention, one or more therapeutically effective cycles of treatment may be administered to a subject. By "therapeutically effective cycle of treatment" is meant a cycle of treatment that when administered, brings about the desired therapeutic response (i.e., reduction in oral-esophageal pain) with respect to treatment. For example, one or more therapeutically effective cycles of treatment may be necessary to reduce the oral-esophageal pain to a manageable level, such as by reducing oral-esophageal pain as reported by the subject by 1 pain rating or more, such as 2 pain ratings or more, such as 3 pain ratings or more and including 4 pain ratings or more on a pain rating scale as described above. For example, one or more therapeutically effective cycles of treatment may be necessary to reduce the oral-esophageal pain from a pain rating of 5 to a pain rating of 4 or less, such as a pain rating of 3 or less, such as a pain rating of 2 or less, and including reducing oral-esophageal pain from a pain rating of 5 to a pain rating of 1.

In some embodiments, subjects treated by methods of the invention exhibit a positive therapeutic response. By "positive therapeutic response" is meant that the subject exhibits a reduction in oral-esophageal pain. For example, a subject exhibiting a positive therapeutic response to methods provided by the invention may include but is not limited to responses such as reduced pain as reported by the subject, reduced inflammation, reduced irritability or a combination thereof.

In certain embodiments, treatment regimens may include multiple dosage intervals. A dosage interval is a single administration of the subject ibuprofen solid oral dosage compositions, beginning with placing the ibuprofen solid oral dosage composition in an oral cavity of the subject and ending with either the removal of the ibuprofen solid oral dosage composition from the oral cavity or complete consumption of the ibuprofen composition in the oral cavity. By "multiple dosage intervals" is meant more than one ibuprofen solid oral dosage composition is administered to the subject in a sequential manner. As such, a first ibuprofen solid oral dosage composition is either removed from or completely consumed in the oral cavity of the subject and a new ibuprofen solid oral dosage composition is repositioned in the oral cavity of the subject. In practicing methods of the invention, treatment regimens may include two or more dosing intervals, such as three or more dosing intervals, such as four or more dosing intervals, such as five or more dosing intervals, including ten or more dosing intervals.

The duration between dosage intervals in a multiple dosage interval treatment regimen may vary, depending on the physiology of the subject or by the treatment regimen as determined by a health care professional. In certain instances, the duration between dosage intervals in a multiple dosage treatment regimen may be predetermined and follow at regular intervals. As such, the time between dosing intervals may vary and may be 0.5 hours or longer, such as 1 hour or longer, such as 2 hours or longer, such as 4 hours or longer, such as 8 hours or longer, such as 12 hours or longer, such as 16 hours or longer, such as 24 hours or longer, such as 48 hours or longer and including 72 hours or longer. In other instances, the duration between dosage intervals may depend on inflammation as determined by a health care professional during the time the ibuprofen solid oral dosage composition is not in contact with the subject between dosage intervals. For example, a subsequent dosage interval may commence if inflammation increases between dosage intervals. In yet other instances, the duration between dosage intervals may depend on the amount of oral-esophageal pain reported by the subject during the time the ibuprofen solid oral dosage composition is not in contact with the subject between dosage intervals. For example, a subsequent dosage interval may commence when the reported pain reaches a particular threshold. In these instances, a pain threshold for applying a subsequent ibuprofen solid oral dosage composition may be when the subject reports and increase in 1 pain rating or more, such as 2 pain ratings or more, such as 3 pain ratings or more and including 4 pain ratings or more on a pain rating scale as described above. For example, a subsequent dosage interval may commence when the reported oral-esophageal pain increases from a pain rating of 1 to a pain rating of 2 or more, such as a pain rating of 3 or more, such as a pain rating of 4 or more, and including when the oral-esophageal pain increase from a pain rating of 1 to a pain rating of 5..

The location in the oral cavity for administering subsequent ibuprofen solid oral dosage compositions in multiple dosage treatment regimens may be the same or different from the location in the oral cavity where the previous ibuprofen solid oral dosage composition was placed. For example, if a first ibuprofen solid oral dosage composition is applied and maintained in the buccal cavity, one or more subsequent ibuprofen compositions may be reapplied to the same position in the buccal cavity. On the other hand, if a first ibuprofen solid oral dosage composition was applied and maintained in the buccal cavity, one or more subsequent ibuprofen compositions may be reapplied to a different position, such as on top of the tongue or sublingually. Subsequent dosages applied in multiple dosage interval regimens may have the same or different number of ibuprofen-methacrylic acid copolymer solid oral dosage compositions. In certain instances, a subsequent dosage interval in a treatment regimen may contain a higher or lower number of ibuprofen-methacrylic acid copolymer solid oral dosage compositions than the previous dosage interval. For example, the number of ibuprofen-methacrylic acid copolymer compositions may be increased in subsequent dosage intervals by 1 solid oral dosage composition or greater, such as 2 solid oral dosage compositions or greater and including 3 solid oral dosage compositions or greater. On the other hand, the number of ibuprofen-methacrylic acid copolymer solid oral dosage compositions may be decreased in subsequent dosage intervals, such as by 1 solid oral dosage composition or greater, such as 2 solid oral dosage compositions or greater and including 3 solid oral dosage compositions or greater.

In other instances, a subsequent dosage interval may contain a different formulation of ibuprofen than the previous dosage interval, such as different flavors, souring agents, sweeteners, etc. as described in detail above.

### KITS

Also provided are kits, where kits at least include one or more, e.g., a plurality of, the subject organoleptically acceptable ibuprofen solid oral dosage compositions, as described above and claimed. In certain embodiments, the subject solid oral dosage compositions in the kits may be provided in a package. For example, the solid oral dosage compositions of the kits may be presented in individual pouches, bottles, or analogous containers, to preserve the solid oral dosage compositions until use. For example, one form of suitable packaging is a blister pack of a water-impermeable plastics material (e.g., polyvinylchloride) closed by a metallic e.g., aluminium foil. In practicing methods according to certain embodiments as described above, the subject removes the solid oral dosage composition by applying pressure to the blister to force the solid oral dosage composition to rupture and pass through the metal foil seal.

Kits may further include other components for practicing the subject methods, such as administration devices (e.g., solid oral dosage composition applicator) or fluids to rinse the oral cavity before administering one or more of the subject solid oral dosage compositions. Kits may also include gauze pads or other devices for cleaning the oral cavity, etc. which may find use in practicing the subject methods.

In addition, kits may also include instructions for how to use the subject organoleptically acceptable ibuprofen solid oral dosage compositions, where the instructions may include information about to how administer the solid oral dosage composition, dosing schedules, and record keeping devices for executing a treatment regimen. The instructions are recorded on a suitable recording medium. For example, the instructions may be printed on a substrate, such as paper or plastic, etc. As such, the instructions may be present in the kits as a package insert, in the labeling of the container of the kit or components thereof (i.e. associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, the protocol for obtaining the instructions may be recorded on a suitable substrate.

### UTILITY

The above described formulations and methods find use in any application in which the administration of ibuprofen to a subject, particularly to treat oral-esophageal pain or inflammation, is desired. The subject methods as described herein are effective for treating inflammation, aches, including the maladies of the oral-esophageal region, such as sore throat, hoarse voice, among other conditions.

In some embodiments, the subject methods find use in the treatment of a sore throat, throat inflammation, oral pain, laryngeal pain, upper and lower neck pain or esophageal inflammation. In yet other embodiments, the subject methods find use in the treatment of hoarse voice, such from extended periods of voice use, speaking, singing, etc. The above described compositions, kits and methods provide at least some amelioration of pain or inflammation in a subject, where amelioration is used in a broad sense to refer to at least a reduction in the magnitude of pain. As such, treatment also includes situations where the pain is completely inhibited, *e.g*. prevented from happening, or stopped, *e*.*g*. terminated, such that the host no longer suffers from the pain. As such, treatment includes both curing and managing a pain.

Aspects of the invention further appear in the following clauses.
1. A solid oral dosage composition as claimed comprising:
   ibuprofen; and
   a methacrylic acid copolymer in an amount sufficient to make the solid oral dosage composition organoleptically acceptable for administering in an oral cavity of a subject.
2. The solid oral dosage composition according to Clause 1, wherein the methacrylic acid copolymer comprises the formula: 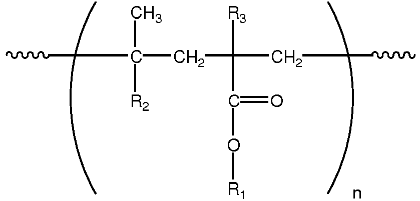
3. The solid oral dosage composition according to Clause 2, wherein R₁ is ethyl, R₂ is a carboxylic acid.
4. The solid oral dosage composition according to Clause 2, wherein R₃ is H.
5. The solid oral dosage composition according to any of Clauses 1 to 4, wherein the amount of ibuprofen in the solid oral dosage composition ranges from 20 mg to 200 mg.
6. The solid oral dosage composition according to Clause 5, wherein the amount of ibuprofen in the solid oral dosage composition is 20 mg.
7. The solid oral dosage composition according to Clause 5, wherein the amount of ibuprofen in the solid oral dosage composition is 100 mg.
8. The solid oral dosage composition according to any of the preceding clauses, wherein the amount of methacrylic acid copolymer ranges from 40 mg to 400 mg.
9. The solid oral dosage composition according to Clause 8, wherein the amount of methacrylic acid copolymer is 40 mg.
10. The solid oral dosage composition as claimed according to any of the preceding clauses, wherein the solid oral dosage composition is formulated for maintaining in an oral cavity of a subject for a predetermined amount of time to deliver ibuprofen to the subject.
11. The solid oral dosage composition according to Clause 10, wherein the solid oral dosage composition is formulated to be maintained in the oral cavity of a subject for 15 minutes or more.
12. The solid oral dosage composition according to Clause 11, wherein the solid oral dosage composition releases 3% or more of the ibuprofen into the oral cavity in 10 minutes or more.
13. The solid oral dosage composition according to Clause 10, wherein the solid oral dosage composition is formulated to be disintegrated in the oral cavity by chewing.
14. The solid oral dosage composition according to Clause 10, wherein the solid oral dosage composition is formulated to be dissolved in the oral cavity.
15. The solid oral dosage composition according to any of the preceding clauses, wherein the solid oral dosage composition further comprises a souring agent.
16. The solid oral dosage composition according to Clause 15, wherein the souring agent is an organic acid.
17. The solid oral dosage composition according to Clause 16, wherein the organic acid is selected from the group consisting of citric acid, asparaginic acid, malic acid, glutamic acid, maleic acid, oxalic acid, taurine, tartaric acid and succinic acid.
18. The solid oral dosage composition according to any of the preceding clauses, wherein the solid oral dosage composition further comprises a buffer.
19. The solid oral dosage composition according to Clause 18, wherein the buffer is a salt.
20. The solid oral dosage composition according to Clause 19, where the salt is selected from the group consisting of sodium citrate, sodium acetate, sodium tartrate, sodium succinate, sodium maleate and sodium phosphate.
21. The solid oral dosage composition according to any of the preceding clauses, wherein the solid oral dosage composition further comprises a diluent, a binder and a lubricant.
22. The solid oral dosage composition according to Clause 21, wherein the diluent is a compound selected from the group consisting of lactose, fructose, starch syrup, reduced malt sugar, D-mannitol, D-sorbitol, sucrose and xylitol.
23. The solid oral dosage composition according to Clause 21, wherein the lubricant is a compound selected from the group consisting of magnesium stearate, talc, and sucrose fatty acid ester.
24. The solid oral dosage composition according to Clause 21, wherein the diluent is xylitol and the lubricant is magnesium stearate.
25. The solid oral dosage composition according to any of the preceding clauses, wherein the solid oral dosage composition further comprises a sweetener.
26. The solid oral dosage composition according to Clause 25, wherein the sweetener is selected from the group consisting of sucralose, sucrose, liquid glucose, acesulfam, glycerol, sorbitol, saccharin, a glycyrrhizin-based sweetener and aspartame.
27. The solid oral dosage composition according to any of the preceding clauses, where the solid oral dosage composition further comprises a flavoring agent.
28. The solid oral dosage composition according to Clause 27, wherein the flavoring agent is a flavor selected from the group consisting of menthol, WS-23, orange, lemon, lime, lemon-lime, grapefruit and tangerine.
29. The solid oral dosage composition according to any of the preceding clauses, wherein the solid oral dosage composition further comprises a disintegrant.
30. The solid oral dosage composition according to Clause 29, wherein the disintegrant is a compound selected from the group consisting of pre-gelatinized starch, carmellose calcium, hydrated silicon dioxide, croscarmellose sodium, microcrystalline cellulose, low substituted hydroxypropylcellulose (L-HPC), corn starch, potato starch, partly pre-gelatinized starch and crospovidone.
31. The solid oral dosage composition according to any of the preceding clauses, wherein the solid oral dosage composition does not include a cyclodextrin.
32. A solid oral dosage composition as claimed consisting of:
   ibuprofen;
   a methacrylic acid copolymer in an amount sufficient to make the solid oral dosage composition organoleptically acceptable for administering in an oral cavity of a subject;
   a diluent;
   a lubricant;
   a souring agent;
   a flavoring agent;
   a binder;
   a sweetener; and
   a buffer.
33. A solid oral dosage composition as claimed consisting of:
   ibuprofen;
   a methacrylic acid copolymer in an amount sufficient to make the solid oral dosage composition organoleptically acceptable for administering in an oral cavity of a subject;
   a diluent;
   a lubricant;
   a souring agent;
   a flavoring agent;
   a binder;
   a sweetener
   a disintegrant; and
   a buffer.
34. A method of making a solid oral dosage composition, as claimed, the method comprising:
   mixing ibuprofen with a methacrylic acid copolymer and a buffer to produce a ibuprofen-methacrylic acid copolymer composition;
   granulating the ibuprofen-methacrylic acid copolymer composition with water to produce a wet ibuprofen-methacrylic acid copolymer granulate;
   drying the wet ibuprofen-methacrylic acid copolymer granulate;
   milling the dried ibuprofen-methacrylic acid copolymer granulate to produce an intermediate ibuprofen-methacrylic acid copolymer granulate composition;
   mixing the intermediate ibuprofen-methacrylic acid copolymer granulate composition with one or more of a diluent, a disintegrant, a binder, a lubricant, a souring agent, a sweetener and a flavoring agent to produce an ibuprofen-methacrylic acid copolymer solid oral dosage composition precursor; and
   tableting the ibuprofen-methacrylic acid copolymer solid oral dosage composition precursor to produce the solid oral dosage composition.
35. The method according to Clause 34, wherein mixing ibuprofen with a methacrylic acid copolymer and a buffer comprises adding each of the ibuprofen, methacrylic acid copolymer and buffer to a mixer in the order:
   1) methacrylic acid copolymer;
   2) buffer; and
   3) ibuprofen.
36. The method according to Clause 35, wherein adding each of the ibuprofen, methacrylic acid copolymer and buffer to the mixer comprises passing the ibuprofen, methacrylic acid copolymer and buffer through a 20 mesh screen.
37. The method according to the Clause 34, 35 or 36, wherein the water has a pH of from 7 to 8.
38. The method according to any of Clauses 34 to 37, wherein the wet ibuprofen-methacrylic acid copolymer granulate is passed through a mesh screen before drying.
39. The method according to Clause 38, wherein the mesh screen is a 10 mesh screen.
40. The method according to any of Clauses 34 to 39, wherein drying the wet ibuprofen-methacrylic acid copolymer granulate comprises drying the granulate at an elevated temperature and under a constant airflow.
41. The method according to Clause 40, wherein the wet ibuprofen-methacrylic acid copolymer granulate is dried at a temperature of from about 50 °C to about 60 °C.
42. The method according to any of Clauses 34 to 41, wherein milling the dried ibuprofen-methacrylic acid copolymer granulate comprises milling the granulate through a 20 mesh screen.
43. The method according to any of Clauses 34 to 42, wherein mixing the intermediate ibuprofen-methacrylic acid copolymer granulate composition with a diluent, a lubricant, a souring agent, a sweetener and a flavoring agent comprises adding each of the ibuprofen-methacrylic acid copolymer the intermediate ibuprofen-methacrylic acid copolymer granulate composition, diluent, binder, souring agent, sweetener and flavoring agent to a mixer in the order:
   1) diluent;
   2) the intermediate ibuprofen-methacrylic acid copolymer granulate composition
   3) flavoring agent;
   4) sweetener;
   5) souring agent; and
   6) binder.
44. The method according to Clause 43, wherein adding each of the diluent, intermediate ibuprofen-methacrylic acid copolymer granulate composition, flavoring agent, sweetener, souring agent and binder to the mixer comprises passing the diluent, the intermediate ibuprofen-methacrylic acid copolymer granulate composition, flavoring agent, sweetener, souring agent and binder through a 20 mesh screen.
45. The method according to Clause 43, wherein the method further comprises adding the lubricant to the mixer after adding the binder.
46. The method according to Clause 45, wherein adding the lubricant to the mixer comprises passing the lubricant through a 40 mesh screen.
47. The method according to any of Clauses 34 to 46, wherein the methacrylic acid copolymer comprises the formula: 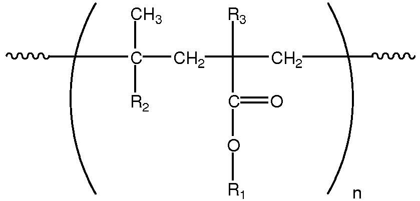
48. The method according to Clause 47, wherein R₁ is ethyl, R₂ is a carboxylic acid.
49. The method according to Clause 47, wherein R₃ is H.
50. The method according to any of Clauses 34 to 49, wherein the method comprises mixing 1:1 to 1:3 mass ratio of ibuprofen to methacrylic acid copolymer to produce the ibuprofen-methacrylic acid copolymer composition.
51. The method according to any of Clauses 34 to 50, wherein the souring agent is an organic acid selected from the group consisting of citric acid, asparaginic acid, malic acid, glutamic acid, taurine, maleic acid, oxalic acid, tartaric acid and succinic acid.
52. The method according to any of Clauses 34 to 51, wherein the buffer is a salt selected from the group consisting of sodium citrate, sodium acetate, sodium tartrate, sodium succinate, sodium maleate and sodium phosphate.
53. The method according to any of Clauses 34 to 52, wherein the diluent is a compound selected from the group consisting of lactose, fructose, starch syrup, reduced malt sugar, D-mannitol, D-sorbitol, sucrose and xylitol.
54. The method according to Clause 53, wherein the diluent is xylitol.
55. The method according to any of Clauses 34 to 54, wherein the disintegrant is a compound selected from the group consisting of pre-gelatinized starch, camellose calcium, hydrated silicon dioxide, croscarmellose sodium, microcrystalline cellulose, low substituted hydroxypropylcellulose (L-HPC), corn starch, potato starch, partly pre-gelatinized starch and crospovidone.
56. The method according to any of Clauses 34 to 55, wherein the lubricant is a compound selected from the group consisting of magnesium stearate, talc, and sucrose fatty acid ester.
57. The method according to any of Clause 34 to 56, wherein the binder is silicified microcrystalline cellulose.
58. The method according to any of Clauses 34 to 57, wherein the lubricant is magnesium stearate.
59. The method according to any of Clauses 34 to 58, wherein the sweetener is a sweetener selected from the group consisting of sucralose, sucrose, liquid glucose, glycerol, sorbitol, saccharin, a glycyrrhizin-based sweetener and aspartame.
60. The method according to any of Clauses 34 to 59, wherein the flavoring agent is a flavor selected from the group consisting of orange, lemon, lime, lemon-lime, grapefruit and tangerine.
61. A solid oral dosage composition prepared by the process of any of Clauses 34 to 60.
62. A method of treating a subject suffering from oral-esophageal pain, the method comprising:
   placing a solid oral dosage claimed composition comprising ibuprofen and a methacrylic acid copolymer into an oral cavity of the subject; and
   maintaining the solid oral dosage composition in the oral cavity of the subject for an amount of time sufficient to treat the subject for oral-esophageal pain.
63. The method according to Clause 62, wherein maintaining the solid oral dosage composition in the oral cavity comprises retaining the solid oral dosage composition in an oral cavity selected from the group consisting of the sublingual cavity, the buccal cavity, on the upper palate, on top of the tongue, and against the gums of the subject.
64. The method according to Clauses 62 or 63, wherein maintaining the solid oral dosage composition in the oral cavity comprises chewing the solid oral dosage composition.
65. The method according to Clause 62, 63 or 64, wherein maintaining the solid oral dosage composition in the oral cavity comprises dissolving the solid oral dosage composition in the oral cavity of the subject.
66. The method according to any of Clauses 62 to 65, wherein the solid oral dosage composition is maintained in the oral cavity for 15 minutes or more.
67. The method according to Clause 66, wherein the solid oral dosage composition releases 3% or more of the ibuprofen into the oral cavity in 10 minutes or more.
68. The method according to any of Clauses 62 to 67, wherein the solid oral dosage composition is completely disintegrated in the oral cavity of the subject.
69. The method according to any of Clauses 62 to 68, wherein maintaining the solid oral dosage composition in the oral cavity comprises sucking on the solid oral dosage composition.
70. The method according to any of Clauses 62 to 69, wherein maintaining the solid oral dosage composition in the oral cavity comprises not biting or chewing the solid oral dosage composition.
71. The method according to any of Clauses 62 to 70, wherein the methacrylic acid copolymer comprises the formula: 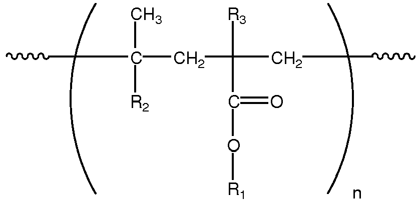
72. The method according to Clause 71, wherein R₁ is ethyl, R₂ is a carboxylic acid.
73. The method according to Clause 71, wherein R₃ is H.
74. The method according to any of Clauses 62 to 73, wherein the amount of ibuprofen in the solid oral dosage composition is from about 20 mg to about 200 mg.
75. The method according to Clause 74, wherein the amount of methacrylic acid copolymer is from about 40 mg to about 400 mg.
76. The method according to any of Clauses 62 to 75, wherein the solid oral dosage composition comprises a souring agent selected from the group consisting of citric acid, asparaginic acid, malic acid, glutamic acid, taurine, maleic acid, oxalic acid, tartaric acid and succinic acid.
77. The method according to any of Clauses 62 to 76, wherein the solid oral dosage composition comprises a buffer selected from the group consisting of sodium citrate, sodium acetate, sodium tartrate, sodium succinate, sodium maleate and sodium phosphate.
78. The method according to any of Clauses 62 to 77, wherein the solid oral dosage composition comprises a diluent selected from the group consisting of lactose, fructose, starch syrup, reduced malt sugar, D-mannitol, D-sorbitol, sucrose and xylitol.
79. The method according to any of Clauses 62 to 78, wherein the solid oral dosage composition comprises a lubricant selected from the group consisting of magnesium stearate, talc, and sucrose fatty acid ester.
80. The method according to any of Clauses 62 to 79, wherein the solid oral dosage composition comprises a sweetener selected from the group consisting of sucralose, sucrose, liquid glucose, glycerol, sorbitol, saccharin, a glycyrrhizin-based sweetener and aspartame.
81. The method according to any of Clauses 62 to 80, wherein the solid oral dosage composition comprises a flavoring agent selected from the group consisting of orange, lemon, lime, lemon-lime, grapefruit and tangerine.
82. The method according to any of Clauses 62 to 81, wherein the solid oral dosage composition does not include a cyclodextrin.
83. The method according to any of Clauses 62 to 82, wherein the oral-esophageal pain is a sore throat.
84. A kit comprising two or more organoleptically acceptable solid oral dosage claimed compositions, wherein the organoleptically acceptable solid oral dosage compositions comprises:
   ibuprofen; and
   a methacrylic acid copolymer in an amount sufficient to make the ibuprofen organoleptically acceptable for administering in an oral cavity of a subject.
85. The kit according to Clause 84, wherein the methacrylic acid copolymer comprises the formula: 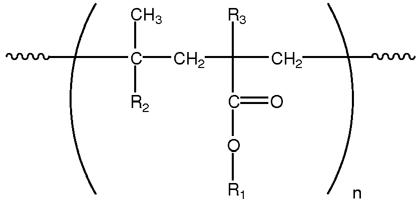
86. The kit according to Clause 85, wherein R₁ is ethyl, R₂ is a carboxylic acid.
87. The kit according to Clause 85, wherein R₃ is H.
88. The kit according to any of Clauses 84 to 87, further comprising a solid oral dosage composition applicator for positioning a solid oral dosage composition in an oral cavity of a subject.
89. The kit according to any of Clauses 84 to 88, further comprising an oral rinse for rinsing the oral cavity of a subject.

The following practical and comparative examples are offered by way of illustration and not by way of limitation.

### EXPERIMENTAL

### Preparing an ibuprofen-methacrylic acid copolymer solid oral dosage composition

A planetary mixer with a mixing bowl and agitator is set up with a peristaltic pump with tubing to delivery about 15-20 g of water per minute. To the mixing bowl of the planetary mixer, ingredients for an ibuprofen-methacrylic acid copolymer composition are passed through a 20 mesh screen and dispensed into the mixing bowl in the order: 1) half amount of methacrylic acid copolymer type C; 2) sodium citrate; 3) ibuprofen; and 4) the remaining half amount of methacrylic acid copolymer type C. The ingredients of the ibuprofen-methacrylic acid copolymer composition are premixed for 3 minutes on stir speed. The ingredients are wet granulated, using pH adjusted water (pH adjusted to a pH of 7.3-7.5 with sodium citrate dihydrate) added to the ibuprofen-methacrylic acid copolymer composition at a rate of 15-20 grams per minute. The ibuprofen-methacrylic acid copolymer is wet granulated at a rate of 15-20 g per minute. The wet granulate is removed from the planetary mixer and passed through a 10 mesh screen. An examples of a wet granulate formulation is summarized in Table 1. After placing the wet screened granulate in an expansion chamber, the wet granulate is air-dried under an airflow of between 40-60% relative humidity and an inlet temperature of 55 °C until a loss on drying (LOD) of ≤ 3.0% is obtained. The dried ibuprofen-methacrylic acid copolymer granulate is milled in a comil through a 20 mesh screen with a round impeller at a speed of about 300 to 350 RPM (revolutions per minute) to produce a milled ibuprofen-methacrylic acid copolymer dry granulate. Pharmaceutical excipients were added to the milled ibuprofen-methacrylic acid copolymer granulate and blended for 15 minutes using a Patterson-Kelley blender. The ingredients for blending the milled ibuprofen-methacrylic acid copolymer granulate were added to the Patterson-Kelley by passing through a 20 mesh screen in the order: 1) half amount of xylitol; 2) the milled ibuprofen-methacrylic acid copolymer granulate; 3) orange flavoring; 4) sucralose; 5) magnasweet MM125; 6) tartaric acid; 7) citric acid; 8) silicified microcrystalline cellulose; and 9) the remaining half amount of xylitol. After blending the components for 15 minutes, magnesium stearate was dispensed into the blender by passing the magnesium stearate through a 40-mesh screen into the mixture. The composition was blended for an additional 5 minutes to produce an ibuprofen-methacrylic acid copolymer solid oral dosage composition precursor. The ibuprofen-methacrylic acid copolymer solid oral dosage composition precursor was tableted into ibuprofen-methacrylic acid copolymer solid oral dosage compositions using a Stokes B2 tablet press. The hardness, friability, thickness, weight variation and disintegration were characterized for at least six or more the produced tablets. Example organoleptically acceptable ibuprofen solid oral dosage composition formulations are summarized below in Tables 2 and 3.

**Table 1 - Ibuprofen-Methacrylic Acid Copolymer Granulation**

| **Component** | **% w/w** | **Batch weight (g)** |
|---|---|---|
| Ibuprofen, USP | 31.25 | 200.0 |
| Methacrylic Acid Copolymer Type C, USP/NF (Eudragit^{®} L100-55) | 62.50 | 400.0 |
| Sodium Citrate Dihydrate, USP | 6.25 | 40.0 |
| Water | n/a | 300 - 350 |

**Table 2 - Example Ibuprofen Solid oral dosage composition Formulation - Ibuprofen 20 mg dosage**

| **Component** | **% w/w** | **mg/unit** | **Batch weight (g)** |
|---|---|---|---|
| Ibuprofen-methacrylic copolymer Granulation (Ibuprofen, USP; Methacrylic Acid Copolymer Type C (Eudragit^{®} L100-55), | (2.50 + 5.00 + 0.5 = 8.00) | (20.00 + 40.00 + 4.00 = 64.00) | 32.00 |
| USP/NF; Sodium Citrate Dihydrate, USP) | 8.00 | 64.00 | |
| Xylitol (Xylitab® 200) | 64.00 | 512.00 | 256.00 |
| Natural & Artificial Orange Flavor | 5.00 | 40.00 | 20.00 |
| Magnasweet | 2.50 | 20.00 | 10.00 |
| Sucralose, NF | 5.00 | 40.00 | 20.00 |
| Silicified Microcrystalline Cellulose | 10.00 | 80.00 | 40.00 |
| Citric Acid, Anhydrous, USP | 1.50 | 12.00 | 6.00 |
| Tartaric Acid, NF | 1.50 | 12.00 | 6.00 |
| Sodium Citrate Dihydrate, USP | 2.00 | 16.00 | 8.00 |
| Magnesium Stearate, NF | 0.50 | 4.00 | 2.00 |

**Table 3 - Example Ibuprofen Solid oral dosage composition Formulation - Ibuprofen 100 mg dosage**

| **Component** | **% w/w** | **mg/unit** | **Batch weight (g)** |
|---|---|---|---|
| Ibuprofen-methacrylic copolymer Granulation (Ibuprofen, USP; Methacrylic Acid Copolymer Type C (Eudragit^{®} L100-55), USP/NF; Sodium Citrate Dihydrate, USP) | (12.50 + 25.00 + 2.5 = 40.00) | (100.00 + 200.00 + 20.00 = 320.00) | 160.00 |
| | 40.00 | 320.00 | |
| Xylitol (Xylitab^{®} 200) | 34.00 | 272.00 | 136.00 |
| Natural & Artificial Orange Flavor | 5.00 | 40.00 | 20.00 |
| Magnasweet | 2.50 | 20.00 | 10.00 |
| Sucralose, NF | 5.00 | 40.00 | 20.00 |
| Silicified Microcrystalline Cellulose | 10.00 | 80.00 | 40.00 |
| Citric Acid, Anhydrous, USP | 1.50 | 12.00 | 6.00 |
| Tartaric Acid, NF | 1.50 | 12.00 | 6.00 |
| Magnesium Stearate, NF | 0.50 | 4.00 | 2.00 |

### Dissolution of Ibuprofen-Methacrylic Acid Copolymer Solid oral dosage compositions

The dissolution of example Ibuprofen-Methacrylic Acid Copolymer Solid oral dosage composition (100 mg were studied using simulated salivary fluid in 100mL of media to simulate dissolution in the mouth. Samples were collected at multiple time points and analyzed using HPLC-UV.

### Dissolution Conditions:

- Dissolution Media: Simulated Salivary Fluid (19mM Phosphate Buffer, 150mM Sodium Chloride, pH 6.75)
- Dissolution Media Volume: 100mL in 100mL vessel
- Mini-paddles, 100 rpm with 250 rpm infinity spin after 120 minutes
- Vessel Temperature: 37 °C ± 0.5 °C
- Pull Volume: 3mL with media replacement
- Sampling Time Points: 5, 10, 15, 30, 60, 120, and 135 minutes

### HPLC Conditions:

- HPLC: Agilent 1100, Xcelience ID 000844
- Column: Waters Atlantis dC18, 4.6 x 50mm, 3µm, Xcelience ID C1171
- Mobile Phase: 50% Water, pH 2.5; 50% Acetonitrile
- Column Temperature: 30°C
- Flow rate: 2.0 mL/min
- Injection Volume: 5µL
- Run Time: 4 minutes

Due to the increased sample concentration relative to the qualified dissolution method, linearity from 0.1 mg/mL to 1 mg/mL was evaluated. The stock solution (1 mg/mL) was prepared using 10% Acetonitrile in dissolution media. All other solutions were prepared in 100% dissolution media. The concentration range evaluated was found to be linear with a correlation coefficient of 1.000. Thus, a standard prepared at 0.5mg/mL was used to quantitate the dissolution samples.

In addition, solubility of ibuprofen was evaluated by adding an excess of ibuprofen to simulated salivary fluid and sonicating for approximately 1 hour. The solution was then centrifuged and injected onto the HPLC. The sample was quantitated against a known standard concentration and found to be 1.8mg/mL. The amount of ibuprofen released from example ibuprofen-methacrylic acid copolymer solid oral dosage compositions by simulated salivary dissolution is summarized in Table 4.

**Table 4 - Percent Ibuprofen release from Ibuprofen-Methacrylic Acid Copolymer Solid oral dosage composition**

| **Vessel** | **5 min** | **10 min** | **15 min** | **30 min** | **60 min** | **120 min** | **135 min** |
|---|---|---|---|---|---|---|---|
| 1 | 1 | 3 | 5 | 8 | 16 | 34 | 76 |
| 2 | 1 | 3 | 5 | 13 | 21 | 40 | 82 |
| 3 | 2 | 3 | 7 | 13 | 21 | 45 | 82 |
| Mean | 1 | 3 | 6 | 11 | 19 | 40 | 80 |

### Accelerated Stability of Ibuprofen-Methacrylic Acid Copolymer Solid oral dosage compositions

Ibuprofen-methacrylic acid copolymer solid oral dosage compositions were evaluated by accelerated stability testing to determine the stability of subject formulations. 20 mg and 100 mg ibuprofen-methacrylic acid copolymer solid oral dosage compositions were evaluated at 60 °C over the course of one week.

Each of the ibuprofen-methacrylic acid copolymer solid oral dosage compositions (formulations summarized in Table 5 were prepared as described above and stability was determined at the initial and 1 week time points for degradation, presence of related substances and visual appearance. (Tables 6 and 7).

**Table 5 -Ibuprofen-Methacrylic Acid Copolymer Solid oral dosage composition Formulation - 100 mg and 20 mg for Accelerated Stability Testing**

| **Component** | **Ibuprofen - Methacrylic Acid Copolymer Solid oral dosage composition Dosage** | | | |
|---|---|---|---|---|
| | **100 mg** | | **20 mg** | |
| | **% w/w** | **mg/unit** | **% w/w** | **mg/unit** |
| Ibuprofen, USP | 12.50 | 100.00 | 2.50 | 20.00 |
| Methacrylic Acid Copolymer Type C, USP/NF (Eudragit^{®} L100-55) | 25.00 | 200.00 | 5.00 | 40.00 |
| Xylitol, USP/NF | 34.00 | 272.00 | 64.00 | 512.00 |
| Natural Orange Flavor | 5.00 | 40.00 | 5.00 | 40.00 |
| Magnasweet MM125 | 2.50 | 20.00 | 2.50 | 20.00 |
| Sucralose | 5.00 | 40.00 | 5.00 | 40.00 |
| Silicified Microcrystalline Cellulose | 10.00 | 80.00 | 10.00 | 80.00 |
| Sodium Citrate Dihydrate, USP | 2.50 | 20.00 | 2.50 | 20.00 |
| Citric Acid, USP | 1.50 | 12.00 | 1.50 | 12.00 |
| Tartaric Acid, NF | 1.50 | 12.00 | 1.50 | 12.00 |
| Magnesium Stearate, NF | 0.50 | 4.00 | 0.50 | 4.00 |

**Table 6 - Accelerated Stability of Ibuprofen-Methacrylic Acid Copolymer Solid oral dosage compositions**

| **Test** | **Dosage = 100 mg** | | **Dosage = 20 mg** | |
|---|---|---|---|---|
| | t = 0 | t = 1 week, 60°C | t = 0 | t = 1 week, 60°C |
| | (% Area) | (% Area) | (% Area) | (% Area) |
| Assay (% LC) | 98.4 | 99.1 | 98.5 | 91.7 |
| Total Impurities | 0.00 | 1.79 | 0.19 | 0.31 |
| Related Compound C (4-isobutylacetophenone) | 0.00 | 0.00 | 0.00 | 0.00 |

**Table 7 - Accelerated Stability of Ibuprofen-Methacrylic Acid Copolymer Solid oral dosage compositions - Visual Appearance**

| **Test** | **Dosage = 100 mg** | | **Dosage** = **20 mg** | |
|---|---|---|---|---|
| | t = 0 | t = 1 week, 60°C | t = 0 | t = 1 week, 60°C |
| | (% Area) | (% Area) | (% Area) | (% Area) |
| Visual Appearance | Round beveled edge, pale mottled yellow tablet with "R&D" debossment on one side | Round beveled edge, off white tablet with "R&D" debossment on one side, brown spots | Round beveled edge, pale mottled yellow tablet with "R&D" debossment on one side, "R&D" had picking | Round beveled edge, off white tablet with "R&D" debossment on one side, brown spots |

As summarized in Tables 6 and 7, the ibuprofen-methacrylic acid copolymer compositions demonstrated excellent stability with few impurities or degradation products produced.

Although the foregoing invention has been described in some detail by way of illustration and example for purposes of clarity of understanding, it is readily apparent to those of ordinary skill in the art in light of the teachings of this invention. Accordingly, the preceding merely illustrates the principles of the invention. It will be appreciated that those skilled in the art will be able to devise various arrangements which, although not explicitly described or shown herein, embody the principles of the invention. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein.

## Claims

1. A solid oral dosage composition comprising ibuprofen and a methacrylic acid copolymer, which composition includes a matrix having methacrylic acid copolymer-ibuprofen polyelectrolyte complexes where ibuprofen is complexed with the methacrylic acid copolymer by ionic interactions,
wherein the mass ratio of ibuprofen to methacrylic acid copolymer is from 1:1 to 1:3, wherein the methacrylic acid copolymer comprises the formula: 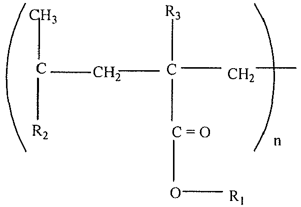 where R₁ is C₂H₅, R₂ is -COOH and R₃ is H, and
wherein the solid oral dosage composition is formulated for maintaining in the oral cavity of a subject for 15 minutes or more to deliver ibuprofen to the subject.

2. The solid oral dosage composition according to claim 1, wherein the amount of ibuprofen in the solid oral dosage composition ranges from 20 mg to 200 mg.

3. The solid oral dosage composition according to either claim 1 or claim 2, wherein the amount of methacrylic acid copolymer ranges from 40 mg to 400 mg.

4. The solid oral dosage composition according to any one of claims 1 to 3, wherein the solid oral dosage composition further comprises a souring agent.

5. The solid oral dosage composition according to any one of claims 1 to 4, wherein the solid oral dosage composition further comprises a buffer.

6. The solid oral dosage composition according to any one of claims 1 to 5, wherein the solid oral dosage composition further comprises a diluent, a binder and a lubricant.

7. The solid oral dosage composition according to any one of claims 1 to 6, wherein the solid oral dosage composition further comprises a sweetener.

8. The solid oral dosage composition according to any one of claims 1 to 7, wherein the solid oral dosage composition further comprises a flavoring agent.

9. The solid oral dosage composition according to any one of claims 1 to 8, wherein the solid oral dosage composition does not include a cyclodextrin.

10. The solid oral dosage composition according to any one of claims 1 to 9, wherein the mass ratio of ibuprofen to the methacrylic acid copolymer is 1:2.

11. The solid oral dosage composition according to any one of claims 1 to 10, wherein the amount of ibuprofen in the solid oral dosage composition is 100 mg.

12. The solid oral dosage composition according to any one of claims 1 to 11, wherein the oral dosage composition is a troche.

13. A method of making a solid oral dosage composition according to any one of claims 1 to 12, the method comprising:
mixing ibuprofen with the methacrylic acid copolymer and a buffer to produce an ibuprofen-methacrylic acid copolymer composition;
granulating the ibuprofen-methacrylic acid copolymer composition with water to produce a wet ibuprofen-methacrylic acid copolymer granulate;
drying the wet ibuprofen-methacrylic acid copolymer granulate;
milling the dried ibuprofen-methacrylic acid copolymer granulate to produce an intermediate ibuprofen-methacrylic acid copolymer granulate composition;
mixing the intermediate ibuprofen-methacrylic acid copolymer granulate composition with one or more of a diluent, a disintegrant, a binder, a lubricant, a souring agent, a sweetener and a flavoring agent to produce an ibuprofen-methacrylic acid copolymer solid oral dosage composition precursor; and
tableting the ibuprofen-methacrylic acid copolymer solid oral dosage composition precursor to produce the solid oral dosage composition.

## Patentansprüche

1. Feste orale Dosierungszusammensetzung, umfassend Ibuprofen und ein Methacrylsäurecopolymer, wobei die Zusammensetzung eine Matrix mit Methacrylsäurecopolymer-Ibuprofen-Polyelektrolytkomplexen umfasst, wobei Ibuprofen durch ionische Wechselwirkungen einen Komplex mit dem Methacrylsäurecopolymer bildet,
wobei das Massenverhältnis von Ibuprofen zu Methacrylsäurecopolymer 1:1 bis 1:3 beträgt, wobei das Methacrylsäurecopolymer die folgende Formel umfasst: 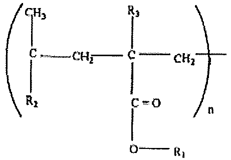 wobei R₁ für C₂H₅ steht, R₂ für -COOH steht, und R₃ für H steht, und
wobei die feste orale Dosierungszusammensetzung formuliert ist, um 15 Minuten oder länger in der Mundhöhle eines Individuums gehalten zu werden, um Ibuprofen an das Individuum abzugeben.

2. Feste orale Dosierungszusammensetzung nach Anspruch 1, wobei die Menge an Ibuprofen in der festen oralen Dosierungszusammensetzung im Bereich von 20 mg bis 200 mg liegt.

3. Feste orale Dosierungszusammensetzung nach Anspruch 1 oder 2, wobei die Menge an Methacrylsäurecopolymer im Bereich von 40 mg bis 400 mg liegt.

4. Feste orale Dosierungszusammensetzung nach einem der Ansprüche 1 bis 3, wobei die feste orale Dosierungszusammensetzung ferner ein Säuerungsmittel umfasst.

5. Feste orale Dosierungszusammensetzung nach einem der Ansprüche 1 bis 4, wobei die feste orale Dosierungszusammensetzung ferner einen Puffer umfasst.

6. Feste orale Dosierungszusammensetzung nach einem der Ansprüche 1 bis 5, wobei die feste orale Dosierungszusammensetzung ferner ein Verdünnungsmittel, ein Bindemittel und ein Gleitmittel umfasst.

7. Feste orale Dosierungszusammensetzung nach einem der Ansprüche 1 bis 6, wobei die feste orale Dosierungszusammensetzung ferner ein Süßungsmittel umfasst.

8. Feste orale Dosierungszusammensetzung nach einem der Ansprüche 1 bis 7, wobei die feste orale Dosierungszusammensetzung ferner einen Aromastoff umfasst.

9. Feste orale Dosierungszusammensetzung nach einem der Ansprüche 1 bis 8, wobei die feste orale Dosierungszusammensetzung kein Cyclodextrin enthält.

10. Feste orale Dosierungszusammensetzung nach einem der Ansprüche 1 bis 9, wobei das Massenverhältnis von Ibuprofen zu Methacrylsäurecopolymer 1:2 beträgt.

11. Feste orale Dosierungszusammensetzung nach einem der Ansprüche 1 bis 10, wobei die Menge an Ibuprofen in der festen oralen Dosierungszusammensetzung 100 mg beträgt.

12. Feste orale Dosierungszusammensetzung nach einem der Ansprüche 1 bis 11, wobei die feste orale Dosierungszusammensetzung eine Pastille ist.

13. Verfahren zur Herstellung einer festen oralen Dosierungszusammensetzung nach einem der Ansprüche 1 bis 12, wobei das Verfahren umfasst:
Mischen von Ibuprofen mit dem Methacrylsäurecopolymer und einem Puffer, um eine Ibuprofen-Methacrylsäurecopolymer-Zusammensetzung zu erzeugen;
Granulieren der Ibuprofen-Methacrylsäurecopolymer-Zusammensetzung mit Wasser, um ein feuchtes Ibuprofen-Methacrylsäurecopolymer-Granulat zu erzeugen;
Trocknen des feuchten Ibuprofen-Methacrylsäurecopolymer-Granulats;
Zermahlen des Ibuprofen-Methacrylsäurecopolymer-Granulats, um eine Ibuprofen-Methacrylsäurecopolymer-Granulat-Zwischenzusammensetzung zu erzeugen;
Mischen der Ibuprofen-Methacrylsäurecopolymer-Granulat-Zwischenzusammensetzung mit einem oder mehreren von Verdünnungsmittel, Aufschlussmittel, Bindemittel, Gleitmittel, Säuerungsmittel, Süßungsmittel und Aromastoff, um ein Vorprodukt einer festen oralen Ibuprofen-Methacrylsäurecopolymer-Dosierungszusammensetzung zu erzeugen; und
Tablettieren des Vorprodukts der festen oralen Ibuprofen-Methacrylsäurecopolymer-Dosierungszusammensetzung, um die feste orale Dosierungszusammensetzung herzustellen.

## Revendications

1. Composition de dosage oral solide comprenant de l'ibuprofène et un copolymère d'acide méthacrylique, laquelle composition inclut une matrice ayant des complexes polyélectrolytes de copolymère d'acide méthacrylique-ibuprofène où l'ibuprofène est complexé avec le copolymère d'acide méthacrylique par des interactions ioniques,
dans laquelle le rapport massique entre l'ibuprofène et le copolymère d'acide méthacrylique est de 1:1 à 1:3, dans laquelle le copolymère d'acide méthacrylique comprend la formule : 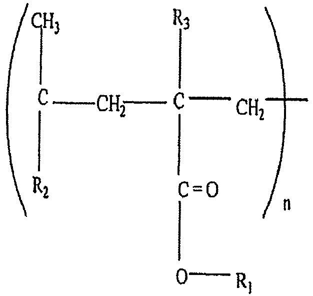 où R₁ est C₂H₅, R₂ est -COOH et R₃ est H, et
dans laquelle la composition de dosage oral solide est formulée pour se maintenir dans la cavité orale d'un sujet pendant 15 minutes ou plus pour délivrer de l'ibuprofène au sujet.

2. Composition de dosage oral solide selon la revendication 1, dans laquelle la quantité d'ibuprofène dans la composition de dosage oral solide varie de 20 mg à 200 mg.

3. Composition de dosage oral solide selon l'une ou l'autre de la revendication 1 ou la revendication 2, dans laquelle la quantité de copolymère d'acide méthacrylique varie de 40 mg à 400 mg.

4. Composition de dosage oral solide selon une quelconque des revendications 1 à 3, dans laquelle la composition de dosage oral solide comprend en outre un agent d'acidification.

5. Composition de dosage oral solide selon une quelconque des revendications 1 à 4, dans laquelle la composition de dosage oral solide comprend en outre un tampon.

6. Composition de dosage oral solide selon une quelconque des revendications 1 à 5, dans laquelle la composition de dosage oral solide comprend en outre un liant et un lubrifiant.

7. Composition de dosage oral solide selon une quelconque des revendications 1 à 6, dans laquelle la composition de dosage oral solide comprend en outre un édulcorant.

8. Composition de dosage oral solide selon une quelconque des revendications 1 à 7, dans laquelle la composition de dosage oral solide comprend en outre un agent aromatisant.

9. Composition de dosage oral solide selon une quelconque des revendications 1 à 8, dans laquelle la composition de dosage oral solide n'inclut pas de cyclodextrine.

10. composition de dosage oral solide selon une quelconque des revendications 1 à 9, dans laquelle le rapport massique entre l'ibuprofène et le copolymère d'acide méthacrylique est de 1:2.

11. Composition de dosage oral solide selon une quelconque des revendications 1 à 10, dans laquelle la quantité d'ibuprofène dans la composition de dosage oral solide est de 100 mg.

12. Composition de dosage oral solide selon une quelconque des revendications 1 à 11, dans laquelle la composition de dosage oral est une tablette.

13. Procédé de fabrication d'une composition de dosage oral solide selon une quelconque des revendications 1 à 12, le procédé consistant à :
mélanger l'ibuprofène avec le copolymère d'acide méthacrylique et un tampon pour produire une composition d'ibuprofène-copolymère d'acide méthacrylique ;
granuler la composition d'ibuprofène-copolymère d'acide méthacrylique avec de l'eau pour produire un granulé humide d'ibuprofène-copolymère d'acide méthacrylique ;
sécher le granulé humide d'ibuprofène-copolymère d'acide méthacrylique ;
moudre le granulé séché d'ibuprofène-copolymère d'acide méthacrylique pour produire une composition de granulé intermédiaire d'ibuprofène-copolymère d'acide méthacrylique ;
mélanger la composition de granulé intermédiaire d'ibuprofène-copolymère d'acide méthacrylique avec un ou plusieurs d'un diluant, un désintégrant, un liant, un lubrifiant, un agent d'acidification, un édulcorant et un agent aromatisant pour produire un précurseur de composition de dosage oral solide d'ibuprofène-copolymère d'acide méthacrylique ; et
mettre sous forme de tablette le précurseur de composition de dosage oral solide d'ibuprofène-copolymère d'acide méthacrylique pour produire la composition de dosage oral solide.
